# EUROPEAN PATENT APPLICATION

(11) **EP 2 703 484 A1**
(43) Date of publication of application: **05.03.2014**
(21) Application number: 12760334.8
(22) Date of filing: 19.03.2012
(51) Int. Cl.: C12N 15/09, C12N 1/19, C12N 9/42, C12P 19/14

(54) **TRANSFORMANT OF YEAST OF GENUS SCHIZOSACCHAROMYCES, METHOD FOR PRODUCING SAME, METHOD FOR PRODUCING BETA-GLUCOSIDASE, AND METHOD FOR DECOMPOSING CELLULOSE**

(30) Priority: 24.03.2011 JP 2011066539
(71) Applicant: Asahi Glass Company, Limited, Tokyo 100-8405 (JP)
(72) Inventor: OKADA, Katsunori, Tokyo 100-8405 (JP); TOHDA, Hideki, Tokyo 100-8405 (JP)
(74) Representative: Blodig, Wolfgang
(86) International application number: PCT/JP2012/057055
(87) International publication number: WO 2012/128260

(57) **Abstract**

To provide a transformant of a yeast of the genus *Schizosaccharomyces* which can produce a modified-type β-glucosidase with improved glucosidase activity, a method for producing such a transformant, a method for producing the modified-type β-glucosidase, and a cellulose degradation method using the modified-type β-glucosidase.

A transformant of a yeast of the genus *Schizosaccharomyces* characterized by having a structural gene sequence comprising a region encoding a modified-type β-glucosidase which is prepared by introducing at least one mutation selected from the group consisting of specific mutations into a β-glucosidase derived from a filamentous fungus, and a promoter sequence and a terminator sequence for expressing the structural gene in a chromosome, or alternatively by having the sequences as an extrachromosomal gene, and is characterized in that the modified-type β-glucosidase has higher thermostability or higher resistance to glucose inhibition than a β-glucosidase which does not have the specific mutations.

SEQUENCE LISTING
<110> ASAHIGLASS COMPANY, LIMITED
<120> Transformant of Yeast of Genus Schizosaccharomyces, Method for Producing
Same, Method for Producing Beta-glucosidase, and Cellulose Degradation Method
<130> F20120092
<160> 33

<210> 1
<211> 860
<212> PRT
<213> Aspergillus aculeatus
<223> BGL1
<400> 1
Met Lys Leu Ser Trp Leu Glu Ala Ala Ala Leu Thr Ala Ala Ser Val
                5                   10                  15
Val Ser Ala Asp Glu Leu Ala Phe Ser Pro Pro Phe Tyr Pro Ser Pro
            20                  25                  30
Trp Ala Asn Gly Gln Gly Glu Trp Ala Glu Ala Tyr Gln Arg Ala Val
        35                  40                  45
Ala Ile Val Ser Gln Met Thr Leu Asp Glu Lys Val Asn Leu Thr Thr
    50                  55                  60
Gly Thr Gly Trp Glu Leu Glu Lys Cys Val Gly Gln Thr Gly Gly Val
65                  70                  75                  80
Pro Arg Leu Asn Ile Gly Gly Met Cys Leu Gln Asp Ser Pro Leu Gly
                85                  90                  95
Ile Arg Asp Ser Asp Tyr Asn Ser Ala Phe Pro Ala Gly Val Asn Val
            100                 105                 110
Ala Ala Thr Trp Asp Lys Asn Leu Ala Tyr Leu Arg Gly Gln Ala Met
        115                 120                 125
Gly Gln Glu Phe Ser Asp Lys Gly Ile Asp Val Gln Leu Gly Pro Ala
    130                 135                 140
Ala Gly Pro Leu Gly Arg Ser Pro Asp Gly Gly Arg Asn Trp Glu Gly
145                 150                 155                 160
Phe Ser Pro Asp Pro Ala Leu Thr Gly Val Leu Phe Ala Glu Thr Ile
                165                 170                 175
Lys Gly Ile Gln Asp Ala Gly Val Val Ala Thr Ala Lys His Tyr Ile
            180                 185                 190
Leu Asn Glu Gln Glu His Phe Arg Gln Val Ala Glu Ala Ala Gly Tyr
        195                 200                 205
Gly Phe Asn Ile Ser Asp Thr Ile Ser Ser Asn Val Asp Asp Lys Thr
    210                 215                 220
Ile His Glu Met Tyr Leu Trp Pro Phe Ala Asp Ala Val Arg Ala Gly
225                 230                 235                 240
Val Gly Ala Ile Met Cys Ser Tyr Asn Gln Ile Asn Asn Ser Tyr Gly
                245                 250                 255
Cys Gln Asn Ser Tyr Thr Leu Asn Lys Leu Leu Lys Ala Glu Leu Gly
            260                 265                 270
Phe Gln Gly Phe Val Met Ser Asp Trp Gly Ala His His Ser Gly Val
        275                 280                 285
Gly Ser Ala Leu Ala Gly Leu Asp Met Ser Met Pro Gly Asp Ile Thr
    290                 295                 300
Phe Asp Ser Ala Thr Ser Phe Trp Gly Thr Asn Leu Thr Ile Ala Val
305                 310                 315                 320
Leu Asn Gly Thr Val Pro Gln Trp Arg Val Asp Asp Met Ala Val Arg
                325                 330                 335
Ile Met Ala Ala Tyr Tyr Lys Val Gly Arg Asp Arg Leu Tyr Gln Pro
            340                 345                 350
Pro Asn Phe Ser Ser Trp Thr Arg Asp Glu Tyr Gly Phe Lys Tyr Phe
        355                 360                 365
Tyr Pro Gln Glu Gly Pro Tyr Glu Lys Val Asn His Phe Val Asn Val
    370                 375                 380
Gln Arg Asn His Ser Glu Val Ile Arg Lys Leu Gly Ala Asp Ser Thr
385                 390                 395                 400
Val Leu Leu Lys Asn Asn Asn Ala Leu Pro Leu Thr Gly Lys Glu Arg
                405                 410                 415
Lys Val Ala Ile Leu Gly Glu Asp Ala Gly Ser Asn Ser Tyr Gly Ala
            420                 425                 430
Asn Gly Cys Ser Asp Arg Gly Cys Asp Asn Gly Thr Leu Ala Met Ala

435                 440                 445
Trp Gly Ser Gly Thr Ala Glu Phe Pro Tyr Leu Val Thr Pro Glu Gln
    450                 455                 460
Ala Ile Gln Ala Glu Val Leu Lys His Lys Gly Ser Val Tyr Ala Ile
465                 470                 475                 480
Thr Asp Asn Trp Ala Leu Ser Gln Val Glu Thr Leu Ala Lys Gln Ala
                485                 490                 495
Ser Val Ser Leu Val Phe Val Asn Ser Asp Ala Gly Glu Gly Tyr Ile
            500                 505                 510
Ser Val Asp Gly Asn Glu Gly Asp Arg Asn Asn Leu Thr Leu Trp Lys
        515                 520                 525
Asn Gly Asp Asn Leu Ile Lys Ala Ala Ala Asn Asn Cys Asn Asn Thr
    530                 535                 540
Ile Val Val Ile His Ser Val Gly Pro Val Leu Val Asp Glu Trp Tyr
545                 550                 555                 560
Asp His Pro Asn Val Thr Ala Ile Leu Trp Ala Gly Leu Pro Gly Gln
                565                 570                 575
Glu Ser Gly Asn Ser Leu Ala Asp Val Leu Tyr Gly Arg Val Asn Pro
            580                 585                 590
Gly Ala Lys Ser Pro Phe Thr Trp Gly Lys Thr Arg Glu Ala Tyr Gly
        595                 600                 605
Asp Tyr Leu Val Arg Glu Leu Asn Asn Gly Asn Gly Ala Pro Gln Asp
    610                 615                 620
Asp Phe Ser Glu Gly Val Phe Ile Asp Tyr Arg Gly Phe Asp Lys Arg
625                 630                 635                 640
Asn Glu Thr Pro Ile Tyr Glu Phe Gly His Gly Leu Ser Tyr Thr Thr
                645                 650                 655
Phe Asn Tyr Ser Gly Leu His Ile Gln Val Leu Asn Ala Ser Ser Asn
            660                 665                 670
Ala Gln Val Ala Thr Glu Thr Gly Ala Ala Pro Thr Phe Gly Gln Val
        675                 680                 685
Gly Asn Ala Ser Asp Tyr Val Tyr Pro Glu Gly Leu Thr Arg Ile Ser
    690                 695                 700
Lys Phe Ile Tyr Pro Trp Leu Asn Ser Thr Asp Leu Lys Ala Ser Ser
705                 710                 715                 720
Gly Asp Pro Tyr Tyr Gly Val Asp Thr Ala Glu His Val Pro Glu Gly
                725                 730                 735
Ala Thr Asp Gly Ser Pro Gln Pro Val Leu Pro Ala Gly Gly Gly Ser
            740                 745                 750
Gly Gly Asn Pro Arg Leu Tyr Asp Glu Leu Ile Arg Val Ser Val Thr
        755                 760                 765
Val Lys Asn Thr Gly Arg Val Ala Gly Asp Ala Val Pro Gln Leu Tyr
    770                 775                 780
Val Ser Leu Gly Gly Pro Asn Glu Pro Lys Val Val Leu Arg Lys Phe
785                 790                 795                 800
Asp Arg Leu Thr Leu Lys Pro Ser Glu Glu Thr Val Trp Thr Thr Thr
                805                 810                 815
Leu Thr Arg Arg Asp Leu Ser Asn Trp Asp Val Ala Ala Gln Asp Trp
            820                 825                 830
Val Ile Thr Ser Tyr Pro Lys Lys Val His Val Gly Ser Ser Ser Arg
        835                 840                 845
Gln Leu Pro Leu His Ala Ala Leu Pro Lys Val Gln
    850                 855                 860

<210> 2
<211> 2664
<212> DNA
<213> Artificial Sequence
<220>
<223> Description of Artificial Sequence: modified AaBGL1 gene
<400> 2
   cgaattggcg gaaggccgtc aaggccgcat ggtacctcat gaagttgtcc tggcttgagg      60
   ccgctgccct taccgccgcc tccgtcgttt ccgccgacga gttggccttc tcccccccct      120
   tctacccctc cccctgggcc aacggtcaag gtgagtgggc cgaggcttac caacgtgccg     180
   tcgccattgt ctcccaaatg acccttgacg agaaggtcaa ccttaccacc ggtactggtt     240
   gggagcttga gaagtgcgtc ggtcaaaccg gtggtgtccc ccgtcttaac atcggtggta     300
   tgtgccttca agactccccc cttggtatcc gtgactccga ctacaactcc gccttccctg     360
   ccggtgtcaa cgtcgccgcc acctgggaca agaaccttgc ctaccttcgt ggtcaagcta     420
   tgggtcaaga gttctccgac aagggtatcg acgtccaact tggtcctgcc gccggtcccc 480
   ttggtcgttc ccccgacggt ggtcgtaact gggagggttt ctcccccgac cccgccctta 540
   ccggtgtcct tttcgccgag actatcaagg gtatccaaga cgctggtgtc gtcgccaccg 600
   ccaagcacta catccttaac gagcaagagc acttccgtca agtcgccgag gccgctggtt 660
   acggtttcaa catctccgac accatctctt ccaacgttga cgacaagacc atccacgaga 720
   tgtacctttg gcccttcgcc gacgccgtcc gtgccggtgt cggtgccatc atgtgctcct 780
   acaaccaaat caacaactcc tacggttgcc aaaactccta cacccttaac aagttgttga 840
   aggccgagct tggtttccaa ggtttcgtca tgtccgactg gggtgcccac cactccggtg 900
   ttggttccgc ccttgccggt cttgacatgt ccatgcccgg tgacatcacc ttcgactccg 960
   ctacctcctt ctggggcacc aaccttacca ttgccgtcct taacggtact gtcccccaat 1020
   ggcgtgttga cgacatggcc gtccgtatca tggccgccta ctacaaggtc ggtcgtgacc 1080
   gtctttacca accccccaac ttctcctcct ggacccgtga cgagtacggt ttcaagtact 1140
   tctaccccca agagggtccc tacgagaagg ttaaccactt cgtcaacgtc caacgtaacc 1200
   actccgaggt catccgtaag ttgggtgccg actccaccgt ccttttgaag aacaacaacg 1260
   ccttgccctt gaccggtaag gagcgtaagg tcgccatcct tggtgaggac gccggttcca 1320
   actcttacgg tgccaacggt tgctccgacc gtggttgcga caacggtact cttgctatgg 1380
   cctggggttc cggtactgcc gagttcccct accttgtcac ccccgagcaa gccatccaag 1440
   ccgaggtctt gaagcacaag ggttccgtct acgccatcac cgacaactgg gccttgtccc 1500
   aagtcgagac tcttgccaag caagcctctg tctcccttgt tttcgtcaac tccgacgccg 1560
   gtgagggtta catctccgtt gacggtaacg agggtgaccg taacaacctt accctttgga 1620
   agaacggtga caaccttatc aaggccgctg ccaacaactg caacaacacc atcgtcgtca 1680
   tccactccgt cggtcccgtc cttgttgacg agtggtacga ccaccccaac gtcaccgcca 1740
   tcctttgggc cggtttgccc ggtcaagagt ccggtaactc ccttgccgac gtcctttacg 1800
   gtcgtgtcaa ccccggtgcc aagtccccct tcacctgggg taagacccgt gaggcttacg 1860
   gtgactacct tgtccgtgag cttaacaacg gtaacggtgc cccccaagac gacttctccg 1920
   agggtgtttt catcgactac cgtggtttcg acaagcgtaa cgagactccc atctacgagt 1980
   tcggtcacgg tttgtcctac accaccttca actactccgg tctccacatc caagtcctta 2040
   acgcctcctc caacgcccaa gtcgccaccg agactggtgc cgcccctacc ttcggtcaag 2100
   tcggtaacgc ctccgactac gtctaccccg agggtcttac ccgtatctcc aagttcatct 2160
   acccctggct taactctacc gacttgaagg cttcctccgg tgacccctac tacggtgttg 2220
   acaccgccga gcacgtcccc gagggtgcca ccgacggttc cccccaaccc gtccttcccg 2280
   ctggtggtgg ttccggtggt aaccctcgtc tttacgacga gcttatccgt gtctccgtca 2340
   ccgtcaagaa caccggtcgt gtcgccggtg acgccgtccc ccaactttac gtttcccttg 2400
   gtggtcccaa cgagcccaag gtcgtccttc gtaagttcga ccgtcttacc ttgaagccct 2460
   ccgaggagac tgtctggacc accaccctta cccgtcgtga cctttccaac tgggacgtcg 2520
   ccgcccaaga ctgggtcatc acctcctacc ccaagaaggt ccacgtcggt tcctcttccc 2580
   gtcaacttcc ccttcacgcc gcccttccca aggtccaatg ataatctaga gctcctgggc 2640
   ctcatgggcc ttccgctcac tgcc 2664

<210> 3
<211> 8515
<212> DNA
<213> Artificial Sequence
<220>
<223> Description of Artificial Sequence: pSL6AaBGL1
<400> 3
cgtacgattt aaatgcggcc gcttcggctg cggcgagcgg gtatcagctc actcaaaggc 60
ggtaatacgg ttatccacag aatcagggga taacgcagga aagaacatgt gagcaaaagg 120
ccagcaaaag gccaggaacc gtaaaaaggc cgcgttgctg gcgtttttcc ataggctccg 180
cccccctgac gagcatcaca aaaatcgacg ctcaagtcag aggtggcgaa acccgacagg 240
actataaaga taccaggcgt ttccccctgg aagctccctc gtgcgctctc ctgttccgac 300
cctgccgctt accggatacc tgtccgcctt tctcccttcg ggaagcgtgg cgctttctca 360
atgctcacgc tgtaggtatc tcagttcggt gtaggtcgtt cgctccaagc tgggctgtgt 420
gcacgaaccc cccgttcagc ccgaccgctg cgccttatcc ggtaactatc gtcttgagtc 480
caacccggta agacacgact tatcgccact ggcagcagcc actggtaaca ggattagcag 540
agcgaggtat gtaggcggtg ctacagagtt cttgaagtgg tggcctaact acggctacac 600
tagaaggaca gtatttggta tctgcgctct gctgaagcca gttaccttcg gaaaaagagt 660
tggtagctct tgatccggca aacaaaccac cgctggtagc ggtggttttt ttgtttgcaa 720
gcagcagatt acgcgcagaa aaaaaggatc tcaagaagat cctttgatct tttctacggg 780
gtctgacgct cagtggaacg aaaactcacg ttaagggatt ttggtcatga gcttgcgccg 840
tcccgtcaag tcagcgtaat gctctgccag tgttacaacc aattaaccaa ttctgattag 900
aaaaactcat cgagcatcaa atgaaactgc aatttattca tatcaggatt atcaatacca 960
tatttttgaa aaagccgttt ctgtaatgaa ggagaaaact caccgaggca gttccatagg 1020
atggcaagat cctggtatcg gtctgcgatt ccgactcgtc caacatcaat acaacctatt 1080
aatttcccct cgtcaaaaat aaggttatca agtgagaaat caccatgagt gacgactgaa 1140
tccggtgaga atggcaagag cttatgcatt tctttccaga cttgttcaac aggccagcca 1200
ttacgctcgt catcaaaatc actcgcatca accaaaccgt tattcattcg tgattgcgcc 1260
tgagcgaggc gaaatacgcg atcgctgtta aaggacaatt acaaacagg aatcgaatgc 1320

aaccggcgca ggaacactgc cagcgcatca acaatatttt cacctgaatc aggatattct 1380
tctaatacct ggaatgctgt tttcccaggg atcgcagtgg tgagtaacca tgcatcatca 1440
ggagtacgga taaaatgctt gatggtcgga agaggcataa attccgtcag ccagtttagt 1500
ctgaccatct catctgtaac atcattggca acgctacctt tgccatgttt cagaaacaac 1560
tctggcgcat cgggcttccc atacaatcgg tagattgtcg cacctgattg cccgacatta 1620
tcgcgagccc atttataccc atataaatca gcatccatgt tggaatttaa tcgcggcctc 1680
gtcgagcaag acgtttcccg ttgaatatgg ctcataacac cccttgtatt actgtttatg 1740
taagcagaca gttttattgt tcatttaaat gcggccgcgt acggcggctt cgatagcttc 1800
agcctcctta ggagcattca aaccataacg aaggagaagg gaagcagata aaattgtacc 1860
aacaggatta acaatgccct tgccagcgat atcgggagcg ctaccgtgaa tgggctcaac 1920
caaacaatga acctttcttt ctgattttcc taccacaccg gaaagggagg cagaaggcaa 1980
aaggcccaag ctaccaggaa tgacagaagc ctcatctgaa ataatgtcac caaacaagtt 2040
gtcagtcaaa acaacaccgt taagtgtacg agggctcttg accaaaagca tggctgcgga 2100
gtcaatgagc tggtttttta aggtaaggtg aggatattcc tccttaaaaa tcttagctac 2160
agtcttgcgc caaagacgag aagttgccaa aacattagct ttgtcgagta atgtgacggg 2220
agcaggaggg ttggaagttt cagctaacca agcagccaaa cgagcaatac gagaaacttc 2280
ttccaaactg taaggccaag tgtccatagc ataacccgat ccgttgtcct cagtgcgctc 2340
accaaagtaa caacctccag taagttctcg tacaacacaa aaatcgacac cttcaacgat 2400
ttcaggcttc aaagggctgt acttgactaa agacttgctg gcaaagttgc aaggtcgaag 2460
gttggcccaa acacccatac tcttacgaag cttcaataaa ccttgctcag gacgacaatt 2520
ggggttggtc cattcaggac caccaacggc acccaaaaga acaccgtcag cttccaaaca 2580
agccttcaca gtctcgtcag tcaaaggggt tccataggca tcaatagagg cacctccaat 2640
cttgtgttct tcaaactcga gttttaactc aggtcgcttc ttctcaacga ctttcaaaac 2700
ctccaaggca gaagcaacaa tttcagggcc aatatggtct cctggtaaga cgacgatttt 2760
ctttgcacac atgttgttga agaagttttg ttgtgaaatg gtttcgtgaa agtttcagac 2820
cctaccgcaa aaatgcctgg tttcgggaaa ctcaacactg ttgcactttt tatactacag 2880
attgggatat cgataatatt gcgtaaaaaa tccttttttt aaaaagcttg tttacagtaa 2940
cgtaaatgac cagaaatcag atgaaaatca caagaaagca aataattcac gttaaatcct 3000
gatatgtttg attttgtgat gaaatcatgg atgttcatag gaattgttga aattgcgctt 3060
ttttaacgaa atatacaagt atcctggagc ttacttaatt aattaatgaa tctttgttcc 3120
taggcccggg ctagtaatca attacggggt cattagttca tagcccatat atggagttcc 3180
gcgttacata acttacggta aatggcccgc ctggctgacc gcccaacgac ccccgcccat 3240
tgacgtcaat aatgacgtat gttcccatag taacgccaat agggactttc cattgacgtc 3300
aatgggtgga gtatttacgg taaactgccc acttggcagt acatcaagtg tatcatatgc 3360
caagtacgcc ccctattgac gtcaatgacg gtaaatggcc cgcctggcat tttgcccagt 3420
acatgacctt atgggacttt cctacttggc agtacatcta cgtattagtc atcgctatta 3480
ccatggtgat gcggttttgg cagtacatca atgggcgtgg atagcggttt gactcacggg 3540
gatttccaag tctccacccc attgacgtca atgggagttt gttttggcac caaaatcaac 3600
gggactttcc aaaatgtcgt aacaactccg ccccattgac gcaaatgggc ggtaggcgtg 3660
tacggtggga ggtctatata agcagatttc tctttagttc tttgcaagaa ggtagagata 3720
aagacacttt ttcaaacatg aagttgtcct ggcttgaggc cgctgccctt accgccgcct 3780
ccgtcgtttc cgccgacgag ttggccttct cccccccctt ctacccctcc ccctgggcca 3840
acggtcaagg tgagtgggcc gaggcttacc aacgtgccgt cgccattgtc tcccaaatga 3900
ccttgacgga gaaggtcaac cttaccaccg gtactggttg ggagcttgag aagtgcgtcg 3960
gtcaaaccgg tggtgtcccc cgtcttaaca tcggtggtat gtgccttcaa gactcccccc 4020
ttggtatccg tgactccgac tacaactccg ccttccctgc cggtgtcaac gtcgccgcca 4080
cctgggacaa gaaccttgcc taccttcgtg gtcaagctat gggtcaagag ttctccgaca 4140
agggtatcga cgtccaactt ggtcctgccg ccggtcccct tggtcgttcc cccgacggtg 4200
gtcgtaactg ggagggtttc tcccccgacc ccgcccttac cggtgtcctt ttcgccgaga 4260
ctatcaaggg tatccaagac gctggtgtcg tcgccaccgc caagcactac atccttaacg 4320
agcaagagca cttccgtcaa gtcgccgagg ccgctggtta cggtttcaac atctccgaca 4380
ccatctcttc caacgttgac gacaagacca tccacgagat gtacctttgg cccttcgccg 4440
acgccgtccg tgccggtgtc ggtgccatca tgtgctccta caaccaaatc aacaactcct 4500
acggttgcca aaactcctac acccttaaca agttgttgaa ggccgagctt ggtttccaag 4560
gtttcgtcat gtccgactgg ggtgcccacc actccggtgt tggttccgcc cttgccggtc 4620
ttgacatgtc catgcccggt gacatcacct tcgactccgc tacctccttc tggggcacca 4680
accttaccat tgccgtcctt aacggtactg tcccccaatg gcgtgttgac gacatggccg 4740
tccgtatcat ggccgcctac tacaaggtcg gtcgtgaccg tctttaccaa ccccccaact 4800
tctcctcctg gacccgtgac gagtacggtt tcaagtactt ctacccccaa gagggtccct 4860
acgagaaggt taaccacttc gtcaacgtcc aacgtaacca ctccgaggtc atccgtaagt 4920
tgggtgccga ctccaccgtc cttttgaaga acaacaacgc cttgcccttg accggtaagg 4980
agcgtaaggt cgccatcctt ggtgaggacg ccggttccaa ctcttacggt gccaacggtt 5040
gctccgaccg tggttgcgac aacggtactc ttgctatggc ctggggttcc ggtactgccg 5100
agttccccta ccttgtcacc cccgagcaag ccatccaagc cgaggtcttg aagcacaagg 5160
gttccgtcta cgccatcacc gacaactggg ccttgtccca agtcgagact cttgccaagc 5220
aagcctctgt ctcccttgtt ttcgtcaact ccgacgccgg tgagggttac atctccgttg 5280
acggtaacga gggtgaccgt aacaacctta ccctttggaa gaacggtgac aaccttatca 5340
aggccgctgc caacaactgc aacaacacca tcgtcgtcat ccactccgtc ggtcccgtcc 5400

ttgttgacga gtggtacgac caccccaacg tcaccgccat cctttgggcc ggtttgcccg 5460
gtcaagagtc cggtaactcc cttgccgacg tcctttacgg tcgtgtcaac cccggtgcca 5520
agtccccctt cacctggggt aagacccgtg aggcttacgg tgactacctt gtccgtgagc 5580
ttaacaacgg taacggtgcc ccccaagacg acttctccga gggtgttttc atcgactacc 5640
gtggtttcga caagcgtaac gagactccca tctacgagtt cggtcacggt ttgtcctaca 5700
ccaccttcaa ctactccggt ctccacatcc aagtccttaa cgcctcctcc aacgcccaag 5760
tcgccaccga gactggtgcc gcccctacct tcggtcaagt cggtaacgcc tccgactacg 5820
tctaccccga gggtcttacc cgtatctcca agttcatcta cccctggctt aactctaccg 5880
acttgaaggc ttcctccggt gacccctact acggtgttga caccgccgag cacgtccccg 5940
agggtgccac cgacggttcc ccccaacccg tccttcccgc tggtggtggt tccggtggta 6000
accctcgtct ttacgacgag cttatccgtg tctccgtcac cgtcaagaac accggtcgtg 6060
tcgccggtga cgccgtcccc caactttacg tttcccttgg tggtcccaac gagcccaagg 6120
tcgtccttcg taagttcgac cgtcttacct tgaagccctc cgaggagact gtctggacca 6180
ccacccttac ccgtcgtgac ctttccaact gggacgtcgc cgcccaagac tgggtcatca 6240
cctcctaccc caagaaggtc cacgtcggtt cctcttcccg tcaacttccc cttcacgccg 6300
cccttcccaa ggtccaatga taatctagag tcgacctgca ggcatgcaag cttaaatagg 6360
aaagtttctt caacaggatt acagtgtagc tacctacatg ctgaaaaata tagcctttaa 6420
atcattttta tattataact ctgtataata gagataagtc cattttttaa aaatgttttc 6480
cccaaaccat aaaaccctat acaagttgtt ctagtaacaa tacatgagaa agatgtctat 6540
gtagctgaaa ataaaatgac gtcacaagac gatctgcctc gcgcgtttcg gtgatgacgg 6600
tgaaaacctc tgacacatgc agctcccgga gacggtcaca gcttgtctgt aagcggatgc 6660
cgggagcaga caagcccgtc agggcgcgtc agcgggtgtt ggcgggtgtc ggggcgcagc 6720
catgacccag tcacgtagcg atagcggagc ccgggcacta gtgaattcga gtatgtgtac 6780
gagttgtctt taaacccaca gaggtagaat gtatatataa aattaataag ctaagtgtaa 6840
tacttaaaaa atacattaat tggaactcgt atcctaccat ttacaatgtt catccaattt 6900
tttcagattg tactgtaaat agcgtttgaa aacaccaaat tttagaagct aatcactctc 6960
atcataatcg tctacatcct catcgttatc gacgataaaa gaatcatctt gcatgctggg 7020
ttcatccatg ctatcaaacg agggatcaac gtaaataggt gttttcactg tagccgctgc 7080
tcttctggtt ggcctctttc taatcggaga atctgaatct tctggtggct ctgcgttagt 7140
cgaactagct tttggagttg aactactacc tggaataata aaatcatcat cgtcatcttc 7200
aggtgattgt ttctttaccg agcttgcttt tttcccttta ttcttcgcag aagccttcgt 7260
ggatgttatg gtggaaggtt tcaaactgct aggcaacaaa tcatcttcat cgtctgaaga 7320
aaatatggta gtagcaactg gtttattagt ctttcttcct cttccagacg ccgaggctgc 7380
tatttttttg acgggttttt tactacctgc gtcttcagag tcaacagatt gacttctttt 7440
tcttgatttt ccactatcac tgctatccaa tcccgggctc ttagatatgc gatttcttc 7500
aactgataag ccatgagagt tatcctctgt cttgacaatg tttatgtcag atgatttctc 7560
aggttctttc gacgctgcga actcaagtaa agtttgttgc tttcgatttg ttgtagatgg 7620
tttggattcg ctgctagctt ctttttaac agcagtactt gaggaggatc cggcaatagc 7680
cctgggtttc ctagtaccag tggatttacc tcgaggcttc tttttcgttc gatttacaaa 7740
atctcttgag gattgctctt cttctaacat ttctctctga atatcatcca taaccttatt 7800
ccaagcatgc tcaaatgcat ccaaatcatg aagccacaat tctttaggag tttttttaat 7860
caaagcatcc agttcggcca ttacttcgtc ctttttcttg agaagttcca cataccgttc 7920
ataggtcaaa gaccataaag gcattgaaag aaggtaattg taggcatctg aatcctcgtc 7980
ttgcgaaaca tcaccagatt gttcttcttc agcaagagca ttttcaactt ctaaatcaac 8040
caaatgccct ttctttggtt tactgatagg ttgaaacttc ttttccttca gctccacaat 8100
gagatccttt ttcttctttt ttgaaactac aagctccccc tctataatca tatgaataaa 8160
ccgcgcttga tttgaaaatc tatcaaacct tttttccaat tcattaacca tatgctcttt 8220
acgtctctgg tatgtcctta aacgtacttc gtaaaactcg gtcaaaatat cttcaacact 8280
gtcatacttc ttgatccgtc cagatgcatc aaaagcaatc atattactcg ttgcttgagt 8340
acgcgacagt ttaaacttaa cttccaagga ttcatttaat gcttctttca tgccagcttc 8400
ggtaagcgtg acattaaagt gaacatttcc ttcaccgtga tggctttcat agtccacgat 8460
gaatttacga attttttccg taccaacaag accagcctcc agatactcct tcatt 8515

<210> 4
<211> 876
<212> PRT
<213> Artificial Sequence
<220>
<223> Description of Artificial Sequence: P3AaBGL1
<400> 4
Met Lys Ile Thr Ala Val Ile Ala Leu Leu Phe Ser Leu Ala Ala Ala
                5                   10                  15
Ser Pro Ile Pro Val Ala Asp Pro Gly Val Val Ser Val Ser Leu Lys
            20                  25                  30
Lys Arg Ala Asp Glu Leu Ala Phe Ser Pro Pro Phe Tyr Pro Ser Pro
        35                  40                  45
Trp Ala Asn Gly Gln Gly Glu Trp Ala Glu Ala Tyr Gln Arg Ala Val
    50                  55                  60

Ala Ile Val Ser Gln Met Thr Leu Asp Glu Lys Val Asn Leu Thr Thr
65                  70                  75                  80
Gly Thr Gly Trp Glu Leu Glu Lys Cys Val Gly Gln Thr Gly Gly Val
                85                  90                  95
Pro Arg Leu Asn Ile Gly Gly Met Cys Leu Gln Asp Ser Pro Leu Gly
            100                 105                 110
Ile Arg Asp Ser Asp Tyr Asn Ser Ala Phe Pro Ala Gly Val Asn Val
        115                 120                 125
Ala Ala Thr Trp Asp Lys Asn Leu Ala Tyr Leu Arg Gly Gln Ala Met
    130                 135                 140
Gly Gln Glu Phe Ser Asp Lys Gly Ile Asp Val Gln Leu Gly Pro Ala
145                 150                 155                 160
Ala Gly Pro Leu Gly Arg Ser Pro Asp Gly Gly Arg Asn Trp Glu Gly
                165                 170                 175
Phe Ser Pro Asp Pro Ala Leu Thr Gly Val Leu Phe Ala Glu Thr Ile
            180                 185                 190
Lys Gly Ile Gln Asp Ala Gly Val Val Ala Thr Ala Lys His Tyr Ile
        195                 200                 205
Leu Asn Glu Gln Glu His Phe Arg Gln Val Ala Glu Ala Ala Gly Tyr
    210                 215                 220
Gly Phe Asn Ile Ser Asp Thr Ile Ser Ser Asn Val Asp Asp Lys Thr
225                 230                 235                 240
Ile His Glu Met Tyr Leu Trp Pro Phe Ala Asp Ala Val Arg Ala Gly
                245                 250                 255
Val Gly Ala Ile Met Cys Ser Tyr Asn Gln Ile Asn Asn Ser Tyr Gly
            260                 265                 270
Cys Gln Asn Ser Tyr Thr Leu Asn Lys Leu Leu Lys Ala Glu Leu Gly
        275                 280                 285
Phe Gln Gly Phe Val Met Ser Asp Trp Gly Ala His His Ser Gly Val
    290                 295                 300
Gly Ser Ala Leu Ala Gly Leu Asp Met Ser Met Pro Gly Asp Ile Thr
305                 310                 315                 320
Phe Asp Ser Ala Thr Ser Phe Trp Gly Thr Asn Leu Thr Ile Ala Val
                325                 330                 335
Leu Asn Gly Thr Val Pro Gln Trp Arg Val Asp Asp Met Ala Val Arg
            340                 345                 350
Ile Met Ala Ala Tyr Tyr Lys Val Gly Arg Asp Arg Leu Tyr Gln Pro
        355                 360                 365
Pro Asn Phe Ser Ser Trp Thr Arg Asp Glu Tyr Gly Phe Lys Tyr Phe
    370                 375                 380
Tyr Pro Gln Glu Gly Pro Tyr Glu Lys Val Asn His Phe Val Asn Val
385                 390                 395                 400
Gln Arg Asn His Ser Glu Val Ile Arg Lys Leu Gly Ala Asp Ser Thr
                405                 410                 415
Val Leu Leu Lys Asn Asn Asn Ala Leu Pro Leu Thr Gly Lys Glu Arg
            420                 425                 430
Lys Val Ala Ile Leu Gly Glu Asp Ala Gly Ser Asn Ser Tyr Gly Ala
        435                 440                 445
Asn Gly Cys Ser Asp Arg Gly Cys Asp Asn Gly Thr Leu Ala Met Ala
    450                 455                 460
Trp Gly Ser Gly Thr Ala Glu Phe Pro Tyr Leu Val Thr Pro Glu Gln
465                 470                 475                 480
Ala Ile Gln Ala Glu Val Leu Lys His Lys Gly Ser Val Tyr Ala Ile
                485                 490                 495
Thr Asp Asn Trp Ala Leu Ser Gln Val Glu Thr Leu Ala Lys Gln Ala
            500                 505                 510
Ser Val Ser Leu Val Phe Val Asn Ser Asp Ala Gly Glu Gly Tyr Ile
        515                 520                 525
Ser Val Asp Gly Asn Glu Gly Asp Arg Asn Asn Leu Thr Leu Trp Lys
    530                 535                 540
Asn Gly Asp Asn Leu Ile Lys Ala Ala Ala Asn Asn Cys Asn Asn Thr
545                 550                 555                 560
Ile Val Val Ile His Ser Val Gly Pro Val Leu Val Asp Glu Trp Tyr
                565                 570                 575
Asp His Pro Asn Val Thr Ala Ile Leu Trp Ala Gly Leu Pro Gly Gln
            580                 585                 590
Glu Ser Gly Asn Ser Leu Ala Asp Val Leu Tyr Gly Arg Val Asn Pro
        595                 600                 605

Gly Ala Lys Ser Pro Phe Thr Trp Gly Lys Thr Arg Glu Ala Tyr Gly
    610                 615                 620
Asp Tyr Leu Val Arg Glu Leu Asn Asn Gly Asn Gly Ala Pro Gln Asp
625                 630                 635                 640
Asp Phe Ser Glu Gly Val Phe Ile Asp Tyr Arg Gly Phe Asp Lys Arg
                645                 650                 655
Asn Glu Thr Pro Ile Tyr Glu Phe Gly His Gly Leu Ser Tyr Thr Thr
            660                 665                 670
Phe Asn Tyr Ser Gly Leu His Ile Gln Val Leu Asn Ala Ser Ser Asn
        675                 680                 685
Ala Gln Val Ala Thr Glu Thr Gly Ala Ala Pro Thr Phe Gly Gln Val
    690                 695                 700
Gly Asn Ala Ser Asp Tyr Val Tyr Pro Glu Gly Leu Thr Arg Ile Ser
705                 710                 715                 720
Lys Phe Ile Tyr Pro Trp Leu Asn Ser Thr Asp Leu Lys Ala Ser Ser
                725                 730                 735
Gly Asp Pro Tyr Tyr Gly Val Asp Thr Ala Glu His Val Pro Glu Gly
            740                 745                 750
Ala Thr Asp Gly Ser Pro Gln Pro Val Leu Pro Ala Gly Gly Gly Ser
        755                 760                 765
Gly Gly Asn Pro Arg Leu Tyr Asp Glu Leu Ile Arg Val Ser Val Thr
    770                 775                 780
Val Lys Asn Thr Gly Arg Val Ala Gly Asp Ala Val Pro Gln Leu Tyr
785                 790                 795                 800
Val Ser Leu Gly Gly Pro Asn Glu Pro Lys Val Val Leu Arg Lys Phe
                805                 810                 815
Asp Arg Leu Thr Leu Lys Pro Ser Glu Glu Thr Val Trp Thr Thr Thr
            820                 825                 830
Leu Thr Arg Arg Asp Leu Ser Asn Trp Asp Val Ala Ala Gln Asp Trp
        835                 840                 845
Val Ile Thr Ser Tyr Pro Lys Lys Val His Val Gly Ser Ser Ser Arg
    850                 855                 860
Gln Leu Pro Leu His Ala Ala Leu Pro Lys Val Gln
865                 870                 875

<210> 5
<211> 8563
<212> DNA
<213> Artificial Sequence
<220>
<223> Description of Artificial Sequence: pSL6P3AaBGL1
<400> 5
ctctgccagt gttacaacca attaaccaat tctgattaga aaaactcatc gagcatcaaa      60
tgaaactgca atttattcat atcaggatta tcaataccat atttttgaaa aagccgtttc     120
tgtaatgaag gagaaaactc accgaggcag ttccatagga tggcaagatc ctggtatcgg     180
tctgcgattc cgactcgtcc aacatcaata caacctatta atttcccctc gtcaaaaata     240
aggttatcaa gtgagaaatc accatgagtg acgactgaat ccggtgagaa tggcaagagc     300
ttatgcattt ctttccagac ttgttcaaca ggccagccat tacgctcgtc atcaaaatca     360
ctcgcatcaa ccaaaccgtt attcattcgt gattgcgcct gagcgaggcg aaatacgcga     420
tcgctgttaa aaggacaatt acaaacagga atcgaatgca accggcgcag gaacactgcc     480
agcgcatcaa caatattttc acctgaatca ggatattctt ctaatacctg gaatgctgtt     540
ttcccaggga tcgcagtggt gagtaaccat gcatcatcag gagtacggat aaaatgcttg     600
atggtcggaa gaggcataaa ttccgtcagc cagtttagtc tgaccatctc atctgtaaca     660
tcattggcaa cgctaccttt gccatgtttc agaaacaact ctggcgcatc gggcttccca     720
tacaatcggt agattgtcgc acctgattgc ccgacattat cgcgagccca tttataccca     780
tataaatcag catccatgtt ggaatttaat cgcggcctcg tcgagcaaga cgtttcccgt     840
tgaatatggc tcataacacc ccttgtatta ctgtttatgt aagcagacag ttttattgtt     900
catttaaatg cggccgcgta cggcggcttc gatagcttca gcctccttag gagcattcaa     960
accataacga aggagaaggg aagcagataa aattgtacca acaggattaa caatgccctt    1020
gccagcgata tcgggagcgc taccgtgaat gggctcaacc aaacaatgaa ccttttcttc    1080
tgatttcct accacaccgg aaagggaggc agaaggcaaa aggcccaagc taccaggaat    1140
gacagaagcc tcatctgaaa taatgtcacc aaacaagttg tcagtcaaaa caacaccgtt    1200
aagtgtacga gggctcttga ccaaaagcat ggctgcggag tcaatgagct ggttttttaa     1260
ggtaaggtga ggatattcct ccttaaaaat cttagctaca gtcttgcgcc aaagacgaga    1320
agttgccaaa acattagctt tgtcgagtaa tgtgacggga gcaggagggt tggaagtttc    1380
agctaaccaa gcagccaaac gagcaatacg agaaacttct tccaaactgt aaggccaagt    1440
gtccatagca taacccgatc cgttgtcctc agtgcgctca ccaaagtaac aacctccagt    1500
aagttctcgt acaacacaaa aatcgacacc ttcaacgatt tcaggcttca aagggctgta    1560

cttgactaaa gacttgctgg caaagttgca aggtcgaagg ttggcccaaa cacccatact 1620
cttacgaagc ttcaataaac cttgctcagg acgacaattg gggttggtcc attcaggacc 1680
accaacggca cccaaaagaa caccgtcagc ttccaaacaa gccttcacag tctcgtcagt 1740
caaaggggtt ccataggcat caatagaggc acctccaatc ttgtgttctt caaactcgag 1800
ttttaactca ggtcgcttct tctcaacgac tttcaaaacc tccaaggcag aagcaacaat 1860
ttcagggcca atatggtctc ctggtaagac gacgatttc tttgcacaca tgttgttgaa 1920
gaagttttgt tgtgaaatgg tttcgtgaaa gtttcagacc ctaccgcaaa aatgcctggt 1980
ttcgggaaac tcaacactgt tgcacttttt atactacaga ttgggatatc gataatattg 2040
cgtaaaaaat ccttttttta aaaagcttgt ttacagtaac gtaaatgacc agaaatcaga 2100
tgaaaatcac aagaaagcaa ataattcacg ttaaatcctg atatgtttga ttttgtgatg 2160
aaatcatgga tgttcatagg aattgttgaa attgcgcttt tttaacgaaa tatacaagta 2220
tcctggagct tacttaatta attaatgaat ctttgttcct aggcccgggc tagtaatcaa 2280
ttacggggtc attagttcat agcccatata tggagttccg cgttacataa cttacggtaa 2340
atggcccgcc tggctgaccg cccaacgacc cccgcccatt gacgtcaata atgacgtatg 2400
ttcccatagt aacgccaata gggactttcc attgacgtca atgggtggag tatttacggt 2460
aaactgccca cttggcagta catcaagtgt atcatatgcc aagtacgccc cctattgacg 2520
tcaatgacgg taaatggccc gcctggcatt ttgcccagta catgacctta tgggactttc 2580
ctacttggca gtacatctac gtattagtca tcgctattac catggtgatg cggttttggc 2640
agtacatcaa tgggcgtgga tagcggtttg actcacgggg atttccaagt ctccacccca 2700
ttgacgtcaa tgggagtttg ttttggcacc aaaatcaacg ggactttcca aaatgtcgta 2760
acaactccgc cccattgacg caaatgggcg gtaggcgtgt acggtgggag gtctatataa 2820
gcagatttct ctttagttct ttgcaagaag gtagagataa agacactttt tcaaacatga 2880
agatcaccgc tgtcattgcc cttttattct cacttgctgc tgcctcacct attccagttg 2940
ccgatcctgg tgtggtttca gttagcctta agaagcgtgc cgacgagttg gccttctccc 3000
cccccttcta cccctccccc tgggccaacg gtcaaggtga gtgggccgag gcttaccaac 3060
gtgccgtcgc cattgtctcc caaatgaccc ttgacgagaa ggtcaacctt accaccggta 3120
ctggttggga gcttgagaag tgcgtcggtc aaaccggtgg tgtcccccgt cttaacatcg 3180
gtggtatgtg ccttcaagac tcccccttg gtatccgtga ctccgactac aactccgcct 3240
tccctgccgg tgtcaacgtc gccgccacct gggacaagaa ccttgcctac cttcgtggtc 3300
aagctatggg tcaagagttc tccgacaagg gtatcgacgt ccaacttggt cctgccgccg 3360
gtccccttgg tcgttccccc gacggtggtc gtaactggga gggtttctcc cccgaccccg 3420
cccttaccgg tgtccttttc gccgagacta tcaagggtat ccaagacgct ggtgtcgtcg 3480
ccaccgccaa gcactacatc cttaacgagc aagagcactt ccgtcaagtc gccgaggccg 3540
ctggttacgg tttcaacatc tccgacacca tctcttccaa cgttgacgac aagaccatcc 3600
acgagatgta cctttggccc ttcgccgacg ccgtccgtgc cggtgtcggt gccatcatgt 3660
gctcctacaa ccaaatcaac aactcctacg gttgccaaaa ctcctacacc cttaacaagt 3720
tgttgaaggc cgagcttggt ttccaaggtt tcgtcatgtc cgactggggt gcccaccact 3780
ccggtgttgg ttccgccctt gccggtcttg acatgtccat gcccggtgac atcaccttcg 3840
actccgctac ctccttctgg ggcaccaacc ttaccattgc cgtccttaac ggtactgtcc 3900
cccaatggcg tgttgacgac atggccgtcc gtatcatggc cgcctactac aaggtcggtc 3960
gtgaccgtct ttaccaaccc cccaacttct cctcctggac ccgtgacgag tacggtttca 4020
agtacttcta cccccaagag ggtccctacg agaaggttaa ccacttcgtc aacgtccaac 4080
gtaaccactc cgaggtcatc cgtaagttgg gtgccgactc caccgtcctt ttgaagaaca 4140
acaacgcctt gcccttgacc ggtaaggagc gtaaggtcgc catccttggt gaggacgccg 4200
gttccaactc ttacggtgcc aacggttgct ccgaccgtgg ttgcgacaac ggtactcttg 4260
ctatggcctg gggttccggt actgccgagt tcccctacct tgtcaccccc gagcaagcca 4320
tccaagccga ggtcttgaag cacaagggtt ccgtctacgc catcaccgac aactgggcct 4380
tgtcccaagt cgagactctt gccaagcaag cctctgtctc ccttgttttc gtcaactccg 4440
acgccggtga gggttacatc tccgttgacg gtaacgaggg tgaccgtaac aaccttaccc 4500
tttggaagaa cggtgacaac cttatcaagg ccgctgccaa caactgcaac aacaccatcg 4560
tcgtcatcca ctccgtcggt cccgtccttg ttgacgagtg gtacgaccac cccaacgtca 4620
ccgccatcct ttgggccggt ttgcccggtc aagagtccgg taactccctt gccgacgtcc 4680
tttacggtcg tgtcaacccc ggtgccaagt ccccccttcac ctggggtaag acccgtgagg 4740
cttacggtga ctaccttgtc cgtgagctta acaacggtaa cggtgccccc caagacgact 4800
tctccgaggg tgttttcatc gactaccgtg gtttcgacaa gcgtaacgag actcccatct 4860
acgagttcgg tcacggtttg tcctacacca ccttcaacta ctccggtctc cacatccaag 4920
tccttaacgc ctcctccaac gcccaagtcg ccaccgagac tggtgccgcc cctaccttcg 4980
gtcaagtcgg taacgcctcc gactacgtct accccgaggg tcttacccgt atctccaagt 5040
tcatctaccc ctggcttaac tctaccgact tgaaggcttc ctccggtgac ccctactacg 5100
gtgttgacac cgccgagcac gtccccgagg gtgccaccga cggttccccc caacccgtcc 5160
ttcccgctgg tggtggttcc ggtggtaacc ctcgtcttta cgacgagctt atccgtgtct 5220
ccgtcaccgt caagaacacc ggtcgtgtcg ccggtgacgc cgtcccccaa ctttacgttt 5280
cccttggtgg tcccaacgag cccaaggtcg tccttcgtaa gttcgaccgt cttaccttga 5340
agccctccga ggagactgtc tggaccacca cccttacccg tcgtgacctt tccaactggg 5400
acgtcgccgc ccaagactgg gtcatcacct cctaccccaa gaaggtccac gtcggttcct 5460
cttcccgtca acttcccctt cacgccgccc ttcccaaggt ccaatgataa tctagagtcg 5520
acctgcaggc atgcaagctt aaataggaaa gtttcttcaa caggattaca gtgtagctac 5580
ctacatgctg aaaaatatag cctttaaatc atttttatat tataactctg tataatagag 5640

ataagtccat tttttaaaaa tgttttcccc aaaccataaa accctataca agttgttcta 5700
gtaacaatac atgagaaaga tgtctatgta gctgaaaata aaatgacgtc acaagacgat 5760
ctgcctcgcg cgtttcggtg atgacggtga aaacctctga cacatgcagc tcccggagac 5820
ggtcacagct tgtctgtaag cggatgccgg gagcagacaa gcccgtcagg gcgcgtcagc 5880
gggtgttggc gggtgtcggg gcgcagccat gacccagtca cgtagcgata gcggagcccg 5940
ggcactagtg aattcgagta tgtgtacgag ttgtctttaa acccacagag gtagaatgta 6000
tatataaaat taataagcta agtgtaatac ttaaaaaata cattaattgg aactcgtatc 6060
ctaccattta caatgttcat ccaatttttt cagattgtac tgtaaatagc gtttgaaaac 6120
accaaatttt agaagctaat cactctcatc ataatcgtct acatcctcat cgttatcgac 6180
gataaaagaa tcatcttgca tgctgggttc atccatgcta tcaaacgagg gatcaacgta 6240
aataggtgtt ttcactgtag ccgctgctct tctggttggc ctctttctaa tcggagaatc 6300
tgaatcttct ggtggctctg cgttagtcga actagctttt ggagttgaac tactacctgg 6360
aataataaaa tcatcatcgt catcttcagg tgattgtttc tttaccgagc ttgctttttt 6420
ccctttattc ttcgcagaag ccttcgtgga tgttatggtg gaaggtttca aactgctagg 6480
caacaaatca tcttcatcgt ctgaagaaaa tatggtagta gcaactggtt tattagtctt 6540
tcttcctctt ccagacgccg aggctgctat ttttttgacg ggttttttac tacctgcgtc 6600
ttcagagtca acagattgac ttcttttttct tgattttcca ctatcactgc tatccaatcc 6660
cgggctctta gatatgcgat tttcttcaac tgataagcca tgagagttat cctctgtctt 6720
gacaatgttt atgtcagatg atttctcagg ttctttcgac gctgcgaact caagtaaagt 6780
ttgttgcttt cgatttgttg tagatggttt ggattcgctg ctagcttctt ttttaacagc 6840
agtacttgag gaggatccgg caatagccct gggtttccta gtaccagtgg atttacctcg 6900
aggcttcttt ttcgttcgat ttacaaaatc tcttgaggat tgctcttctt ctaacatttc 6960
tctctgaata tcatccataa ccttattcca agcatgctca aatgcatcca aatcatgaag 7020
ccacaattct ttaggagttt ttttaatcaa agcatccagt tcggccatta cttcgtcctt 7080
tttcttgaga agttccacat accgttcata ggtcaaagac cataaaggca ttgaaagaag 7140
gtaattgtag gcatctgaat cctcgtcttg cgaaacatca ccagattgtt cttcttcagc 7200
aagagcattt tcaacttcta aatcaaccaa atgccctttc tttggtttac tgataggttg 7260
aaacttcttt tccttcagct ccacaatgag atcctttttc ttcttttttg aaactacaag 7320
ctcccccctct ataatcatat gaataaaccg cgcttgattt gaaaatctat caaacctttt 7380
ttccaattca ttaaccatat gctctttacg tctctggtat gtccttaaac gtacttcgta 7440
aaactcggtc aaaatatctt caacactgtc atacttcttg atccgtccag atgcatcaaa 7500
agcaatcata ttactcgttg cttgagtacg cgacagttta aacttaactt ccaaggattc 7560
atttaatgct tctttcatgc cagcttcggt aagcgtgaca ttaaagtgaa catttccttc 7620
accgtgatgg ctttcatagt ccacgatgaa tttacgaatt ttttccgtac caacaagacc 7680
agcctccaga tactccttca ttcgtacgat ttaaatgcgg ccgcttcggc tgcggcgagc 7740
gggtatcagc tcactcaaag gcggtaatac ggttatccac agaatcaggg gataacgcag 7800
gaaagaacat gtgagcaaaa ggccagcaaa aggccaggaa ccgtaaaaag gccgcgttgc 7860
tggcgttttt ccataggctc cgcccccctg acgagcatca caaaaatcga cgctcaagtc 7920
agaggtggcg aaacccgaca ggactataaa gataccaggc gtttccccct ggaagctccc 7980
tcgtgcgctc tcctgttccg accctgccgc ttaccggata cctgtccgcc tttctccctt 8040
cgggaagcgt ggcgctttct caatgctcac gctgtaggta tctcagttcg gtgtaggtcg 8100
ttcgctccaa gctgggctgt gtgcacgaac cccccgttca gcccgaccgc tgcgccttat 8160
ccggtaacta tcgtcttgag tccaacccgg taagacacga cttatcgcca ctggcagcag 8220
ccactggtaa caggattagc agagcgaggt atgtaggcgg tgctacagag ttcttgaagt 8280
ggtggcctaa ctacggctac actagaagga cagtatttgg tatctgcgct ctgctgaagc 8340
cagttacctt cggaaaaaga gttggtagct cttgatccgg caaacaaacc accgctggta 8400
gcggtggttt ttttgtttgc aagcagcaga ttacgcgcag aaaaaaagga tctcaagaag 8460
atcctttgat cttttctacg gggtctgacg ctcagtggaa cgaaaactca cgttaaggga 8520
ttttggtcat gagcttgcgc cgtcccgtca agtcagcgta atg 8563

<210> 6
<211> 29
<212> DNA
<213> Artificial Sequence
<220>
<223> Description of Artificial Sequence: mut fw2 primer
<400> 6
ctctttagtt ctttgcaaga aggtagaga 29

<210> 7
<211> 21
<212> DNA
<213> Artificial Sequence
<220>
<223> Description of Artificial Sequence: mut rv3 primer
<400> 7
atgcctgcag gtcgactcta g 21

<210> 8
<211> 21
<212> DNA
<213> Artificial Sequence
<220>
<223> Description of Artificial Sequence: leu fw1 primer
<400> 8
cctcgtcgag caagacgttt c                                                21

<210> 9
<211> 33
<212> DNA
<213> Artificial Sequence
<220>
<223> Description of Artificial Sequence: leu rv1 primer
<400> 9
catgtttgaa aaagtgtctt tatctctacc ttc                                   33

<210> 10
<211> 19
<212> DNA
<213> Artificial Sequence
<220>
<223> Description of Artificial Sequence: top fw3 primer
<400> 10
cgcccttccc aaggtccaa                                                   19

<210> 11
<211> 23
<212> DNA
<213> Artificial Sequence
<220>
<223> Description of Artificial Sequence: top rv1 primer
<400> 11
ctgtggataa ccgtattacc gcc                                              23

<210> 12
<211> 23
<212> DNA
<213> Artificial Sequence
<220>
<223> Description of Artificial Sequence: leu fw3 primer
<400> 12
cggcttcgat agcttcagcc tcc                                              23

<210> 13
<211> 25
<212> DNA
<213> Artificial Sequence
<220>
<223> Description of Artificial Sequence: top rv5 primer
<400> 13
ttcgcaagac gaggattcag atgcc                                            25

<210> 14
<211> 30
<212> DNA
<213> Artificial Sequence
<220>
<223> Description of Artificial Sequence: leu1 primer
<400> 14
cctcacctcc caaatcgcga gtataaagac                                       30

<210> 15
<211> 30
<212> DNA
<213> Artificial Sequence
<220>

<223> Description of Artificial Sequence: top2 primer
<400> 15
agctagcagc gaatccaaac catctacaac                                        30

<210> 16
<211> 43
<212> DNA
<213> Artificial Sequence
<220>
<223> Description of Artificial Sequence: P3AaBGL1 fw primer
<400> 16
ggtttcagtt agccttaaga agcgtgccga cgagttggcc ttc                         43

<210> 17
<211> 27
<212> DNA
<213> Artificial Sequence
<220>
<223> Description of Artificial Sequence: P3AaBGL1 rv primer
<400> 17
ctgcaggtcg actctagatt atcattg                                           27

<210> 18
<211> 32
<212> DNA
<213> Artificial Sequence
<220>
<223> Description of Artificial Sequence: G298X rv primer
<220>
<221> misc_feature
<222> (25)..(26)
<223> n is a, c, g, or t
<400> 18
gaaccaacac cggagtggtg ggcsnnccag tc                                     32

<210> 19
<211> 33
<212> DNA
<213> Artificial Sequence
<220>
<223> Description of Artificial Sequence: G298X fw primer
<220>
<221> misc_feature
<222> (25)..(26)
<223> n is a, c, g, or t
<400> 19
caaggtttcg tcatgtccga ctggnnsgcc cac                                    33

<210> 20
<211> 33
<212> DNA
<213> Artificial Sequence
<220>
<223> Description of Artificial Sequence: V519X rv primer
<220>
<221> misc_feature
<222> (26)..(27)
<223> n is a, c, g, or t
<400> 20
gtaaccctca ccggcgtcgg agttsnngaa aac                                    33

<210> 21
<211> 33
<212> DNA
<213> Artificial Sequence
<220>
<223> Description of Artificial Sequence: V519X fw primer
<220>

<221> misc_feature
<222> (25)..(26)
<223> n is a, c, g, or t
<400> 21
caagcctctg tctcccttgt tttcnnsaac tcc                                    33

<210> 22
<211> 30
<212> DNA
<213> Artificial Sequence
<220>
<223> Description of Artificial Sequence: Q142X rv primer
<220>
<221> misc_feature
<222> (23)..(24)
<223> n is a, c, g, or t
<400> 22
ggagaactct[?]@tgacccatag[?]@csnnaccacg                                    3

<210> 23
<211> 33
<212> DNA
<213> Artificial Sequence
<220>
<223> Description of Artificial Sequence: Q142X fw primer
<220>
<221> misc_feature
<222> (25)..(26)
<223> n is a, c, g, or t
<400> 23
aagaaccttgg cctaccttcg tggtnnsgct atg                                    33

<210> 24
<211> 27
<212> DNA
<213> Artificial Sequence
<220>
<223> Description of Artificial Sequence: Q142X rv primer
<220>
<221> misc_feature
<222> (19)..(20)
<223> n is a, c, g, or t
<400> 24
gtaggggaac tcggcagtsn nggaacc                                    27

<210> 25
<211> 24
<212> DNA
<213> Artificial Sequence
<220>
<223> Description of Artificial Sequence: G468X fw primer
<220>
<221> misc_feature
<222> (16)..(17)
<223> n is a, c, g, or t
<400> 25
atggcctggg gttccnnsac tgcc                                    24

<210> 26
<211> 24
<212> DNA
<213> Artificial Sequence
<220>
<223> Description of Artificial Sequence: Q156L rv primer
<400> 26
gcggcaggac caagtaggac gtcg                                    24

<210> 27

<211> 27
<212> DNA
<213> Artificial Sequence
<220>
<223> Description of Artificial Sequence: Q156L fw primer
<400> 27
caagggtatc gacgtcctac ttggtcc                                          27

<210> 28
<211> 26
<212> DNA
<213> Artificial Sequence
<220>
<223> Description of Artificial Sequence: I260F rv primer
<400> 28
ggttgtagga gcacatgaag gcaccg                                           26

<210> 29
<211> 24
<212> DNA
<213> Artificial Sequence
<220>
<223> Description of Artificial Sequence: I260F fw primer
<400> 29
tgccggtgtc ggtgccttca tgtg                                             24

<210> 30
<211> 25
<212> DNA
<213> Artificial Sequence
<220>
<223> Description of Artificial Sequence: N458S rv primer
<400> 30
gccatagcaa gagtaccgct gtcgc                                            25

<210> 31
<211> 25
<212> DNA
<213> Artificial Sequence
<220>
<223> Description of Artificial Sequence: N458S fw primer
<400> 31
gaccgtggtt gcgacagcgg tactc                                            25

<210> 32
<211> 29
<212> DNA
<213> Artificial Sequence
<220>
<223> Description of Artificial Sequence: G468X fw primer
<400> 32
ccgttaccgt tgttaagctc aaggacaag                                        29

<210> 33
<211> 31
<212> DNA
<213> Artificial Sequence
<220>
<223> Description of Artificial Sequence: G468X fw primer
<400> 33
gcttacggtg actaccttgt ccttgagctt a                                     31

## Description

### TECHNICAL FIELD

The present invention relates to a transformant of a yeast of the genus *Schizosaccharomyces,* a method for producing the transformant, a method for producing fl-glucosidase by using the transformant, and a cellulose degradation method using the transformant. Specifically, the present invention relates to a transformant of a yeast of the genus *Schizosaccharomyces* which can produce a modified-type β-glucosidase having higher thermostability or higher resistance to glucose inhibition than a wild-type β-glucosidase, and a method for producing such a transformant.

### BACKGROUND ART

To produce biomass fuels from a cellulosic biomass such as a wood, rice straw, rice husk and weed including a sugar as a fermentation feedstock, and a bioethanol, etc., it is required to degrade the main structural component of a plant cell wall, cellulose. To degrade cellulose, an acid saccharification method such as a concentrated sulfuric acid saccharification method or a dilute sulfuric acid saccharification method, an enzymatic saccharification method, etc. may be employed. Because of recent development in biotechnology, research and development of an enzymatic saccharification method are actively carried out. In the enzymatic saccharification of cellulose, a group of enzymes generally known as cellulases are utilized. Firstly, an endoglucanase (EG), which has an activity to cleave cellulose chains at random, degrades an amorphous region of cellulose to expose terminal glucose residues. The exposed glucose residues are degraded by a cellobiohydrolase (CBH) to release cellobiose. Thereafter, the released cellobiose is degraded by β-glucosidase (BGL) to release glucose.

For the saccharification of cellulose, filamentous fungi of the genus *Aspergillus* and the genus *Trichoderma* are widely used, since they can produce various cellulases and hemicellulases which are required for decomposing and saccharifying a crystalline cellulose, and they can secrete a large amount of such enzymes to their extracellular environment.

Further, it has been tried to express such cellulases of filamentous fungi in a heterologous microorganism. Non-patent document 1 discloses that a budding yeast *Saccharomyces cerevisiae* was transformed with a gene encoding β-glucosidase 1 (BGL 1) of *Aspergillus aculeatus* to obtain a transformant, and the obtained transformant expressed such an enzyme.

On the other hand, the genetic analysis of a yeast of the genus *Schizosaccharomyces* is more advanced than that of a filamentous fungus of the genus *Aspergillus* or the genus *Trichoderma,* and the yeast has a lot of advantages like availability of various useful mutant strains and gene transfer vectors, and its suitability for industrial large-scale production of a protein. However, the yeast of the genus *Schizosaccharomyces* does not have endogenous β-glucosidase gene, whereby it cannot utilize cellobiose.

Further, development of a highly functional β-glucosidase has been desired. For example, in the enzymatic saccharification method, as the enzymatic hydrolysis of cellulose proceeds, glucose accumulates in the reaction system and the accumulated glucose inhibits β-glucosidase, whereby accumulation of cellobiose proceeds. Further, there is a problem such that the complete degradation of cellulose may not be achieved since the accumulated cellobiose inhibits endoglucanase and cellobiohydrolase. Accordingly, development of a β-glucosidase having higher resistance to glucose inhibition (i.e. less susceptible to glucose inhibition) has been desired.

Further, in general, there is a tendency for an enzymatic reaction such that the higher the reaction temperature, the higher the reaction efficiency. Therefore, by increasing the temperature during cellulose degradation reaction, the efficiency of the cellulose degradation reaction can be increased. Furthermore, as the thermostability increases, it becomes possible to continue enzymatic reaction for a longer period of time. Accordingly, development of a β-glucosidase having higher thermostability has been desired.

Random mutagenesis of an enzyme is one of important techniques for improving the function of the enzyme. By using an error-prone PCR (polymerase chain reaction) method (Non-Patent Document 2), etc., a single-nucleotide substitution is introduced into a target gene in a random manner. By using saturation mutagenesis (Non-Patent Document 2), a codon at a specific site is modified in a random manner. By *in vitro* recombination (Non-Patent Document 2), several mutant genes are recombined to obtain a combination of specific mutation points. By means of the above-described random mutagenesis methods, a random mutation can be introduced into a gene encoding a target enzyme, whereby a mutant enzyme library can be constructed. It is possible to obtain a useful modified enzyme by using a screening method for selecting one having a desired function from the library. Further, useful mutation points can be accumulated by repeating random mutagenesis and screening, whereby functions of the enzyme can be improved further.

However, the improvement of enzyme by random mutagenesis is mostly reported in the cases of *E. coli* (Patent Document 1) and a cell-free protein synthesis system (Patent Document 2), and is not suitable for the mutagenesis of glycoproteins derived from eukaryotes. Further, Patent Document 3 discloses random mutagenesis in budding yeast which requires step to insert a mutant gene into a vector, step to amplify the mutant-gene inserted vector in *E. coli* and step to purify the vector, and which is not simple.

### PRIOR ART DOCUMENTS

### PATENT DOCUMENTS

Patent Document 1: Japanese Patent No. 4402446
Patent Document 2: JP-A-2005-245336
Patent Document 3: Japanese Patent No. 4448772

### NON-PATENT DOCUMENTS

Non-Patent Document 1: G. Tanaka, et al., Biosci. Biotechnol. Biochem., (1998) 62(8), p1615-1618.
Non-Patent Document 2: F. Arnold, et al., "Directed Evolution Library Creation Methods and Protocols", Methods in Molecular Biology, (2003) Vol. 231.

### DISCLOSURE OF INVENTION

### TECHNICAL PROBLEM

Here, the object of the present invention is to provide a transformant of a yeast of the genus *Schizosaccharomyces* which can produce a modified-type β-glucosidase with improved glucosidase activity, a method for producing such a transformant, a method for producing the modified-type β-glucosidase, and a cellulose degradation method using the modified-type β-glucosidase.

### SOLUTION TO PROBLEM

The present inventors have conducted extensive studies to resolve the above-mentioned problems, and as a result, have found that the above-mentioned problems can be resolved by means of a transformant of a yeast the genus *Schizosaccharomyces* having a structural gene encoding a modified-type β-glucosidase which is obtained by introducing a specific mutation into a structural gene sequence encoding a β-glucosidase derived from a filamentous fungus.

That is, the present invention provides the following [1] to [15].
[1] A transformant of a yeast of the genus *Schizosaccharomyces* characterized by having a structural gene sequence comprising a region encoding the following modified-type β-glucosidase, and a promoter sequence and a terminator sequence for expressing the structural gene in a chromosome, or alternatively by having the sequences as an extrachromosomal gene.

Modified-type β-glucosidase: A β-glucosidase derived from a filamentous fungus having at least one mutation selected from the group consisting of the following mutations 1 to 6, and has higher thermostability or higher resistance to glucose inhibition than a β-glucosidase which does not have the mutations.

Mutation 1; a mutation in which an amino acid corresponds to isoleucine at position 244 of AaBGL1 (BGL1 of *Aspergillus aculeatus)* is substituted by phenylalanine.

Mutation 2; a mutation in which an amino acid corresponds to glycine at position 282 of AaBGL1 is substituted by phenylalanine.

Mutation 3; a mutation in which an amino acid corresponds to asparagine at position 442 of AaBGL1 is substituted by serine.

Mutation 4; a mutation in which an amino acid corresponds to glycine at position 452 of AaBGL1 is substituted by serine.

Mutation 5; a mutation in which an amino acid corresponds to valine at position 503 of AaBGL1 is substituted by alanine.

Mutation 6; a mutation in which an amino acid corresponds to arginine at position 613 of AaBGL1 is substituted by leucine.
[2] The transformant of a yeast of the genus *Schizosaccharomyces* according to [1], wherein the β-glucosidase derived from a filamentous fungus is BGL1.
[3] The transformant of a yeast of the genus *Schizosaccharomyces* according to [1] or [2], wherein the filamentous fungus is a microorganism of the genus *Aspergillus.*
[4] The transformant of a yeast of the genus *Schizosaccharomyces* according to any one of Claims 1 to 3, wherein the β-glucosidase derived from a filamentous fungus is comprised of an amino acid sequence having at least 70% homology with an amino acid sequence of SEQ ID NO: 1, and is a protein has a catalytic activity to hydrolyze a β-D-glucopyranoside bond.
[5] The transformant of a yeast of the genus *Schizosaccharomyces* according to any one of [1] to [4], wherein the structural gene sequence further comprises a region encoding a signal or a region encoding a tag at the 5' end side or the 3' end side of the region encoding the modified-type β-glucosidase.
[6] The transformant of a yeast of the genus *Schizosaccharomyces* according to any one of [1] to [5], wherein the structural gene sequence further comprises a region encoding a secretion signal at the 5' end side of the region encoding the modified-type β-glucosidase.
[7] A transformation method for a yeast of the genus *Schizosaccharomyces,* characterized in that the yeast of the genus *Schizosaccharomyces* is transformed by using a vector containing a structural gene sequence comprising a. region encoding the following modified-type β-glucosidase, and a promoter sequence and a terminator sequence for expressing the structural gene.

Modified-type β-glucosidase: A β-glucosidase derived from a filamentous fungus having at least one mutation selected from the group consisting of the following mutations 1 to 6, and has higher thermostability or higher resistance to glucose inhibition than a β-glucosidase which does not have the mutations.

Mutation 1; a mutation in which an amino acid corresponds to isoleucine at position 244 of AaBGL1 (BGL1 of *Aspergillus aculeatus)* is substituted by phenylalanine.

Mutation 2; a mutation in which an amino acid corresponds to glycine at position 282 of AaBGL1 is substituted by phenylalanine.

Mutation 3; a mutation in which an amino acid corresponds to asparagine at position 442 of AaBGL1 is substituted by serine.

Mutation 4; a mutation in which an amino acid corresponds to glycine at position 452 of AaBGL1 is substituted by serine.

Mutation 5; a mutation in which an amino acid corresponds to valine at position 503 of AaBGL1 is substituted by alanine.

Mutation 6; a mutation in which an amino acid corresponds to arginine at position 613 of AaBGL1 is substituted by leucine.
[8] The transformation method for a yeast of the genus *Schizosaccharomyces* according to[7], wherein the structural gene sequence further comprises a region encoding a signal or a region encoding a tag at the 5' end side or the 3' end side of the region encoding the modified-type β-glucosidase.
[9] The transformation method for a yeast of the genus *Schizosaccharomyces* according to [7], wherein the structural gene sequence further comprises a region encoding a secretion signal at the 5' end side of the region encoding the modified-type β-glucosidase.
[10] The transformation method for a yeast of the genus *Schizosaccharomyces* according to any one of [7] to [9], wherein the vector is integrated into at least one position of a chromosome of the yeast of the genus *Schizosaccharomyces.*
[11] The transformation method for a yeast of the genus *Schizosaccharomyces* according to [10], wherein the vector is integrated into a transposon gene Tf2 site of the chromosome.
[12] A method for producing a β-glucosidase, characterized in that the modified-type β-glucosidase is recovered from cells obtained by cultivating the transformant as defined in any one of [1] to [5].
[13] A method for producing a β-glucosidase, characterized in that the modified-type β-glucosidase is recovered from a culture supernatant obtained by cultivating the transformant as defined in [6].
[14] A cellulose degradation method, characterized in that the modified-type β-glucosidase obtained by the production method as defined in [12] or [13] is used.
[15] A cellulose degradation method, characterized in that the transformant as defined in [6] is cultivated in the presence of cellulose.

### ADVANTAGEOUS EFFECTS OF INVENTION

According to the transformation method for a yeast of the genus *Schizosaccharomyces* of the present invention, it is possible to produce a transformant of a yeast of the genus *Schizosaccharomyces* which can produce a modified-type β-glucosidase and is improved at least one of thermostability or resistance to glucose inhibition. Further, the modified-type β-glucosidase recovered from the transformant by the method for producing β-glucosidase of the present invention is suitably used for degradation of cellulose.

### BRIEF DESCRIPTION OF DRAWINGS

[Fig. 1] Fig. 1 shows a phylogenetic tree of β-glucosidases derived from filamentous fungi along with homologies to BGL1 (AaBGL1) encoded by the bgl1 gene of *Aspergillus aculeatus.*
[Fig. 2A] Fig. 2A shows alignments of the amino acid sequence of AaBGL1 and the amino acid sequences of β-glucosidases derived from other filamentous fungi.
[Fig. 2B] Fig. 2B shows alignments of the amino acid sequence of AaBGL1 and the amino acid sequences of β-glucosidases derived from other filamentous fungi.
[Fig. 2C] Fig. 2C shows alignments of the amino acid sequence of AaBGL1 and the amino acid sequences of β-glucosidases derived from other filamentous fungi.
[Fig. 3] Fig. 3 shows a structure map of the expression vector pSL6AaBGL1.
[Fig. 4] Fig. 4 shows a structure map of the expression vector pSL6P3AaBGL1.
[Fig. 5] Fig. 5 shows the test results of Example 8 in which the glucose inhibition and the thermostability at a warming temperature of from 50 to 75°C of the culture supernatants of the respective mutant strains were measured.

### DESCRIPTION OF EMBODIMENTS

β-glucosidase (EC.3.2.1.21) is a generic name of an enzyme which specifically catalyzes hydrolysis of a β-D-glucopyranoside bond. Particularly, it is also called as cellobiase since it degrades cellobiose to glucose, and is widely found in bacteria, filamentous fungi, plants and animals. A plurality of genes encoding β-glucosidase is usually found in each of the species, and for example, existence of bgl1 to bgl7 in a filamentous fungus *Aspergillus oryzae* has been reported (Soy Protein Research, Japan, Vol. 12, pp. 78-83, 2009; and JP-A-2008-086310).

In the present invention and present specification, "β-glucosidase derived from a filamentous fungus" means a wild-type β-glucosidase intrinsic to a filamentous fungus. The filamentous fungus is, among fungi, an eukaryotic microorganism composed of tubular cells called hyphae. As the filamentous fungus, a fungus of the genus *Aspergillus,* the genus *Trichoderma,* the genus *Fusarium,* the genus *Penicillium,* the genus *Acremonium* or the like may, for example, be mentioned. As the filamentous fungus of the genus *Aspergillus, Aspergillus nidulans, Aspergillus oryzae, Aspergillus aculeatus, Aspergillus niger, Aspergillus pulverulentus, Aspergillus terreus* or the like may, for example, be mentioned.

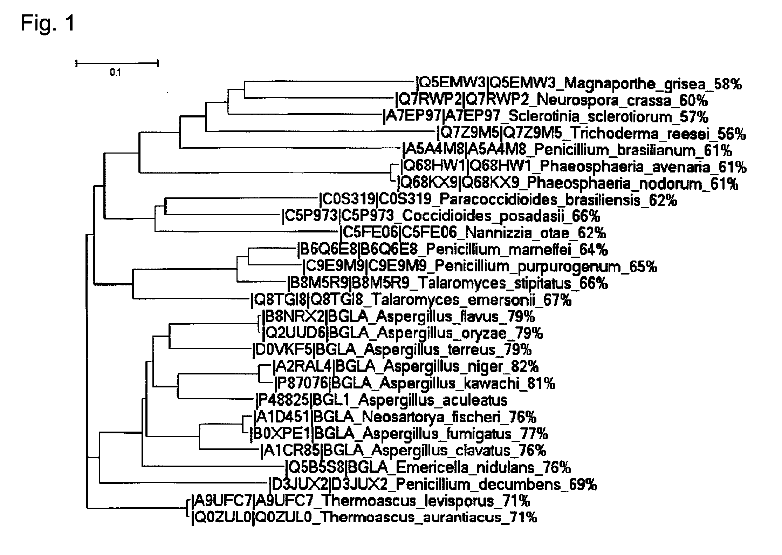

In Fig. 1, a phylogenetic tree of β-glucosidases derived from filamentous fungi is shown along with homologies (%: a value of the terminal of each β-glucosidase) to BGL1 (AaBGL1) encoded by the bgl1 gene of *Aspergillus aculeatus.* Further, in Fig. 1, the first six characters of each β-glucosidase indicate the accession numbers registered in the UniProtKB / Swiss-Prot Database.

In the present specification and present invention, high resistance to glucose inhibition means that the ratio of the activity to catalyze hydrolysis of a β-D-glucopyranoside bond (hereinafter referred to as glucosidase activity) in the presence of glucose to the glucosidase activity in the absence of glucose (i.e. the relative activity value of the glucosidase activity in the presence of glucose, assuming that the glucosidase activity in the absence of glucose is 100%) is high. Further, high thermostability means that the ratio of the glucosidase activity for the case where heating treatment is carried out to the glucosidase activity for the case where heating treatment is not carried out before enzymatic reaction is high.

### [Transformant]

The transformant of the present invention is characterized by having a structural gene sequence comprising a region encoding the following modified-type β-glucosidase, and a promoter sequence and a terminator sequence for expressing the structural gene in a chromosome, or alternatively by having the sequences as an extrachromosomal gene. The modified-type β-glucosidase is a β-glucosidase derived from a filamentous fungus having at least one mutation selected from the group consisting of the below-described mutations 1 to 6, and has higher thermostability or higher resistance to glucose inhibition than a β-glucosidase which does not have the mutations. The modified-type β-glucosidase can be obtained by introducing a specific amino acid substitution mutation into a β-glucosidase derived from a filamentous fungus.

### (Modified-type β-glucosidase)

The modified-type β-glucosidase is an enzyme which has, as compared to a β-glucosidase derived from a filamentous fungus, at least one mutation selected from the group consisting of the following mutations 1 to 6 and has a glucosidase activity. The modified-type β-glucosidase which has mutation 2 or mutation 4 shows higher resistance to glucose inhibition than a β-glucosidase which does not have such mutations. Further, the modified-type β-glucosidase which has mutation 3, mutation 5, or mutation 6 shows higher thermostability than a β-glucosidase which does not have such mutations. The modified-type β-glucosidase which has mutation 1 shows both higher resistance to glucose inhibition and higher thermostability than a β-glucosidase which does not have such mutation.

Mutation 1; a mutation in which an amino acid corresponds to isoleucine at position 244 of AaBGL1 (BGL1 of *Aspergillus aculeatus*) is substituted by phenylalanine.

Mutation 2; a mutation in which an amino acid corresponds to glycine at position 282 of AaBGL1 is substituted by phenylalanine.

Mutation 3; a mutation in which an amino acid corresponds to asparagine at position 442 of AaBGL1 is substituted by serine.

Mutation 4; a mutation in which an amino acid corresponds to glycine at position 452 of AaBGL1 is substituted by serine.

Mutation 5; a mutation in which an amino acid corresponds to valine at position 503 of AaBGL1 is substituted by alanine.

Mutation 6; a mutation in which an amino acid corresponds to arginine at position 613 of AaBGL1 is substituted by leucine.

Further, it was firstly found by the present inventors that thermostability or resistance to glucose inhibition can be increased by having the above-described mutations 1 to 6. Such a finding was obtained by a random mutagenesis using a yeast of the genus *Schizosaccharomyces* as a host and a method for recovering an improved protein via functional screening. Specifically, various mutations were introduced into AaBGL1 by random mutagenesis, and thus obtained AaBGL1 was introduced into a yeast of the genus *Schizosaccharomyces* to construct a mutant-type β-glucosidase library. Thereafter, thus obtained library was screened to select a transformant having improved thermostability or resistance to glucose inhibition, thereby to analyze the nucleotide sequence of a structural gene encoding AaBGL1 of the transformant.

Modified-type β-glucosidase may contain only one type of mutation or at least two types of mutations in combination, among the above-described six types of mutations. For example, a modified-type β-glucosidase which has both mutation 2 and mutation 4 shows higher resistance to glucose inhibition than a β-glucosidase which has only one of them. Further, for example, a modified-type β-glucosidase which has mutation 5 and mutation 6 shows higher thermostability than a modified-type β-glucosidase which has only mutation 5. Further, a modified-type β-glucosidase which has mutation 3, mutation 5 and mutation 6 shows higher thermostability than a modified-type β-glucosidase which has mutation 5 and mutation 6. Further, a modified-type β-glucosidase which has mutation 1, mutation 2, mutation 5 and mutation 6 shows higher thermostability than a modified-type β-glucosidase which has only mutation 1 and mutation 2.

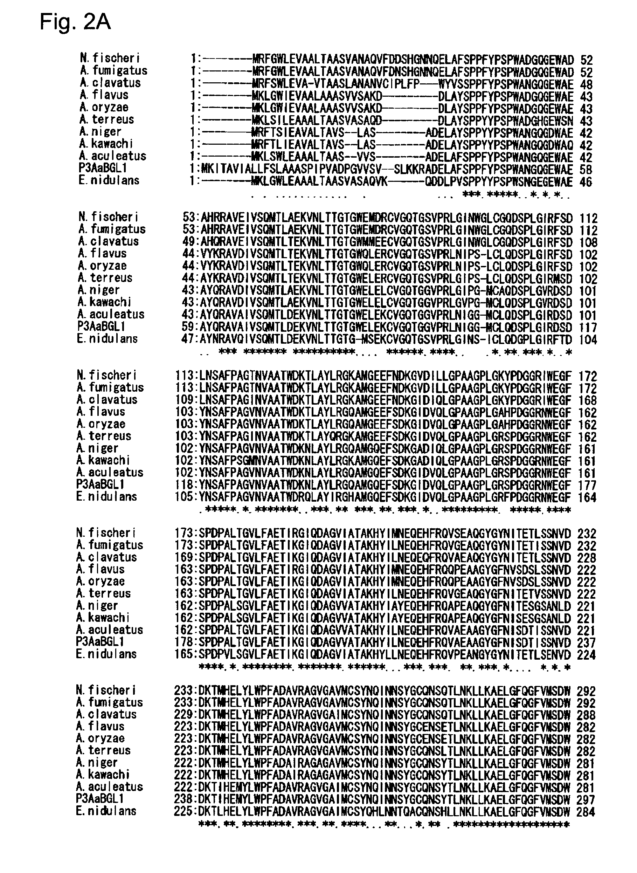

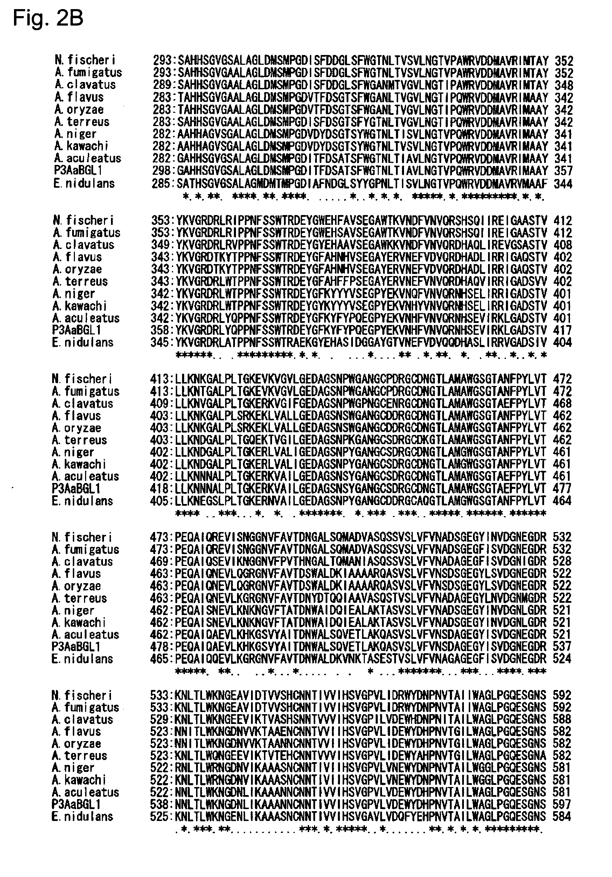

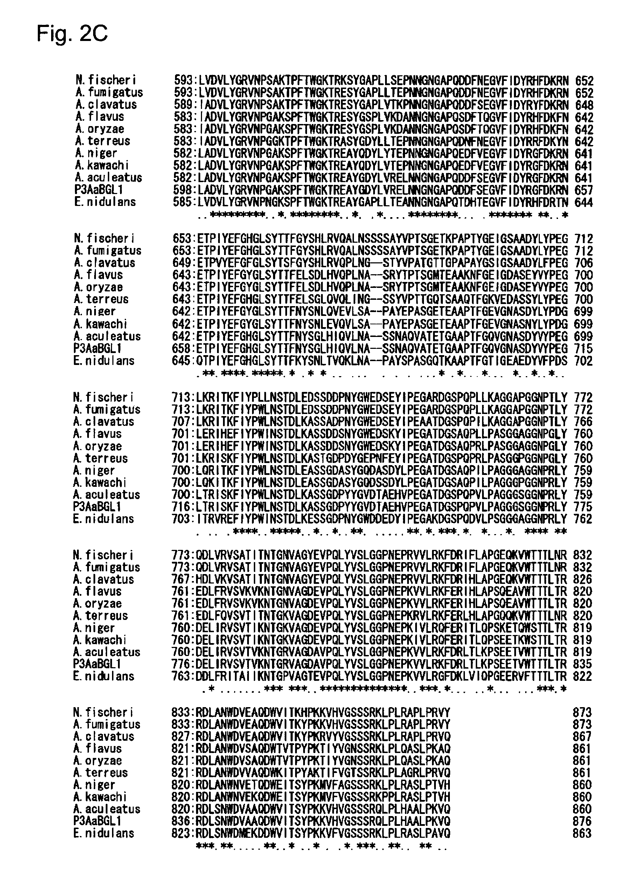

In the present specification and present invention, "amino acid corresponds to amino acid at position X of AaBGL1" in a certain β-glucosidase means an amino acid exists in a position corresponds to an amino acid at position X of AaBGL1, assuming that the amino acid sequence of AaBGL1 (SEQ ID NO: 1) and the amino acid sequence of the β-glucosidase are aligned in a manner to maximize their homology. Fig. 2 shows alignments of the amino acid sequence of AaBGL1 and the amino acid sequences of β-glucosidase derived from other filamentous fungi.

Specifically, as shown in Fig.2, amino acids correspond to isoleucine at position 244 of AaBGL1 are valine at position 245 in *Aspergillus oryzae* or *Aspergillus terreus,* valine at position 244 in *Aspergillus niger,* and isoleucine at position 247 in *Emericella nidulans.*

Further, amino acids correspond to glycine at position 282 of AaBGL1 are threonine at position 283 in *Aspergillus oryzae,* serine at position 283 in *Aspergillus terreus,* alanine at position 282 in *Aspergillus niger,* and serine at position 285 in *Emericella nidulans.*

Further, amino acids correspond to asparagine at position 442 of AaBGL1 are asparagine at position 443 in *Aspergillus oryzae,* lysine at position 443 in *Aspergillus terreus,* asparagine at position 442 in *Aspergillus niger,* and glutamine at position 445 in *Emericella nidulans.*

Further, amino acids correspond to glycine at position 452 of AaBGL1 are glycine at position 453 in *Aspergillus oryzae* or *Aspergillus terreus,* glycine at position 452 in *Aspergillus niger,* and glycine at position 455 in *Emericella nidulans.*

Further, amino acids correspond to valine at position 503 of AaBGL1 are valine at position 504 in *Aspergillus oryzae* or *Aspergillus terreus,* valine at position 503 in *Aspergillus niger,* and valine at position 506 in *Emericella nidulans.*

Further, amino acids correspond to arginine at position 613 of AaBGL1 are lysine at position 614 in *Aspergillus oryzae,* threonine at position 614 in *Aspergillus terreus,* threonine at position 613 in *Aspergillus niger,* and threonine at position 616 in *Emericella nidulans.*

The wild-type β-glucosidase which does not have the mutations of the present may be a protein derived from a filamentous fungus and has a glucosidase activity, but is preferably BGL1 from the viewpoint of its high enzymatic activity. etc.

Further, the wild-type β-glucosidase may be derived from any filamentous fungus so long as it is derived from a filamentous fungus, but is preferably a β-glucosidase derived from a filamentous fungus of the genus *Aspergillus* such as a β-glucosidase derived from *Aspergillus oryzae,* or *Aspergillus aculeatus.* Among them, a β-glucosidase derived from *Aspergillus aculeatus* is preferred since it has high crystalline cellulose degradation ability and high yield of monosaccharide, and AaBGL1 is more preferred. According to the doctoral dissertation of Dr. Reiichiro Sakamoto (Research on cellulase system of *Aspergillus aculeatus* No. F-50, Osaka Prefecture University, 1984), the wild type AaBGL1 purified from *Aspergillus aculeatus* has a molecular weight of about 133 kDa, an optimal pH of 4.0, and a stable pH range of from 3 to 7 (25°C, 24 hours).

The modified-type β-glucosidase of the present invention may, as compared to the wild-type β-glucosidase derived from a filamentous fungus, specifically be one having at least one mutation among the above-described mutations 1 to 6, or one having, in addition to any one of the above-described mutations 1 to 6, other mutations or modifications so long as they do not impair the effects of mutations 1 to 6 and the glucosidase activity. As the modified-type β-glucosidase having such other mutations, etc., one having deletion, substitution or addition of at least one amino acid, preferably from one to tens amino acids, more preferably from one to dozen amino acids, further preferably from one to nine amino acids, even further preferably from one to few amino acids, and having at last one mutation selected from the group consisting of the above-described mutations 1 to 6 in the protein showing a glucosidase activity may, for example, be mentioned. For example, one having deletion of the N-terminal side or C-terminal side region of wild-type β-glucosidase which is comprised of from one to tens amino acids and does not affect glucosidase activity and having at least one mutation selected from the group consisting of the above-described mutations 1 to 6, may be used as the modified-type β-glucosidase.

The modified-type β-glucosidase of the present invention may be one having a homology to the amino acid sequence of SEQ ID NO: 1 of at least 60%, preferably at least 65%, more preferably at least 70%, further preferably at least 75%, even further preferably at least 80%, and having at last one mutation selected from the group consisting of the above-described mutations 1 to 6 in the protein showing a catalytic activity to hydrolyze a β-D-glucopyranoside bond.

### (Host)

The host of the transformant of the present invention is a yeast of the genus *Schizosaccharomyces.* The yeast of the genus *Schizosaccharomyces* to be used in the present invention may be a wild-type or a mutant-type in which a specific gene is deleted or inactivated depending on application. For deletion or inactivation of a specific gene, conventional methods can be used. Specifically, the Latour system (Nucleic Acids Res. (2006) 34: e11, and WO2007/063919) can be used to delete the gene. Further, the gene can be inactivated by mutating the gene at a certain position by mutant screening using mutagens (Koubo Bunshi Idengaku Jikken-Hou, 1996, Japan Scientific Societies Press), random mutations using PCR (polymerase chain reaction) (PCR Methods Appl., 1992, vol. 2, p.28-33) and the like. As the yeast of the genus *Schizosaccharomyces* host in which a specific gene is deleted or inactivated, ones disclosed in WO2002/101038, W02007/015470, etc. may, for example, be used.

Further, it is preferred to use a yeast of the genus *Schizosaccharomyces* host having a marker for selecting a transformant. For example, it is preferred to use a host which essentially requires a specific nutrient factor for growth due to deletion of a gene. When preparing a transformant by using a vector containing a target gene sequence, a transformant lacking the auxotrophy of the host can be obtained by using a vector carrying the deleted gene (auxotrophic complementation marker). It is possible to select the transformant by using the difference in auxotrophy between the host and the transformant.

For example, a yeast of the genus *Schizosaccharomyces* host which has been made auxotrophic for uracil by deletion or inactivation of orotidine 5'-phosphate decarboxylase gene (ura4 gene) is transformed with a vector containing ura4 gene (auxotrophic complementation marker), and transformants carrying the vector are obtained by selecting ones lacking uracil auxotrophy. The gene to be deleted to make an auxotrophic host is not limited to ura4 gene when it is used for selection of a transformant, and may, for example, be isopropyl malate dehydrogenase gene (leu1 gene).

As the yeast of the genus *Schizosaccharomyces, Schizosaccharomyces pombe, Schizosaccharomyces japonicus,* and *Schizosaccharomyces octosporus* may, for example, be mentioned. Among the above-mentioned yeasts of the genus *Schizosaccharomyces, Schizosaccharomyces pombe* is preferred in view of the availability of various useful mutant strains.

### (Expression cassette)

The expression cassette is a combination of DNA essential for expressing a protein, and contains a structural gene sequence encoding the protein, and a promoter and a terminator capable of functioning in yeast of the genus *Schizosaccharomyces.*

The transformant of the present invention is prepared by introducing an expression cassette containing a structural gene sequence comprising a region encoding the above-described modified-type β-glucosidase (modified-type β-glucosidase structural gene sequence), and a promoter sequence and a terminator sequence for expressing the structural gene, into a yeast of the genus *Schizosaccharomyces.*

The expression cassette may additionally contain at least one of a 5'-untranslated region and a 3'-untranslated region. The expression cassette preferably contains all of the modified-type β-glucosidase structural gene sequence, the promoter, the terminator, the 5'-untranslated region and the 3'-untranslated region. Further, genes such as an auxotrophic complementation marker may be contained.

The nucleotide sequence of the region encoding the modified-type β-glucosidase in the modified-type β-glucosidase structural gene sequence is obtained by introducing a nucleotide substitution which reflects at least one amino acid substitution selected from the group consisting of mutations 1 to 6, and other modification as the case requires, into a structural gene of a wild-type β-glucosidase by using a genetic engineering method. Further, a gene encoding β-glucosidase derived from a filamentous fungus may be used as it is for a portion excluding the portion where the above-described modification is introduced. However, to increase expression in yeast of the genus *Schizosaccharomyces,* it is preferred to modify the above-described gene sequence by changing its codons to ones frequently used in a gene highly expressed in yeast of the genus *Schizosaccharomyces.*

The modified-type β-glucosidase structural gene sequence may contain, in addition to a region encoding the modified-type β-glucosidase, a region encoding other peptide or protein. In such a case, the transformant of the present invention produces a protein in which such other peptide is bound to the N-terminal or C-terminal of the modified-type β-glucosidase. As the peptide to be bound to the modified-type β-glucosidase, a signal such as a secretion signal, an organelle localization signal or the like, and a tag such as His-tag or FLAG-tag may, for example, be mentioned. The signal should be a signal capable of functioning in yeast of the genus *Schizosaccharomyces.*

The secretion signal is a peptide introduced at the N-terminal and having a function of secreting the expressed protein out of the host cell. As the secretion signal capable of functioning in yeast of the genus *Schizosaccharomyces,* P3 signal described in WO1996/2389 is particularly preferred.

The modified-type β-glucosidase structural gene sequence is preferably a sequence in which a region encoding a secretion signal is bound to the 5' end side of the region encoding the modified-type β-glucosidase. By having a secretion signal at the N-terminal, it becomes possible to secrete the modified-type β-glucosidase produced in the transformant out of the cells of a yeast of the genus *Schizosaccharomyces.* The modified-type β-glucosidase secreted out of the cells can be easily collected and purified from the supernatant of a culture broth.

The promoter and the terminator may be ones capable of functioning in a yeast of the genus *Schizosaccharomyces* host and can direct expression of the modified-type β-glucosidase. As the promoter capable of functioning in yeast of the genus *Schizosaccharomyces,* a promoter intrinsic to the yeast of the genus *Schizosaccharomyces* (preferably one having a high transcriptional activity) or a promoter extrinsic to the yeast of the genus *Schizosaccharomyces* (such as a promoter derived from a virus) may be used. Further, two or more types of promoters may be contained in the vector.

As the promoter intrinsic to the yeast of the genus *Schizosaccharomyces,* a promoter of alcohol dehydrogenase gene, a promoter of nmt1 gene which relates to thiamine metabolism, a promoter of fructose 1,6-bisphosphatase gene which relates to glucose metabolism, a promoter of an invertase gene which relates to catabolite repression (WO99/23223) or a promoter of a heat shock protein gene (WO2007/26617) may, for example, be mentioned.

As the promoter extrinsic to the yeast of the genus *Schizosaccharomyces,* a promoter derived from an animal cell virus disclosed in JP-A-5-15380, JP-A-7-163373 or JP-A-10-234375 may, for example, be mentioned, and hCMV promoter or SV40 promoter is preferred.

As the terminator capable of functioning in yeast of the genus *Schizosaccharomyces,* a terminator intrinsic to the yeast of the genus *Schizosaccharomyces,* or a terminator extrinsic to the yeast of the genus *Schizosaccharomyces,* may be used. Further, two or more types of terminators may be contained in the vector.

As the terminator, the terminator derived from the human disclosed in JP-A-5-15380, JP-A-7-163373 or JP-A-10-234375 may, for example, be mentioned, and human lipocortin-I terminator is preferred.

### [Transformation Method]

The transformation method for a yeast of the genus *Schizosaccharomyces* of the present invention (hereinafter referred to as transformation method of the present invention) is characterized in that the yeast of the genus *Schizosaccharomyces* is transformed by using a vector containing the above-described expression cassette.

The yeast of the genus *Schizosaccharomyces* to be used as a host, modified-type β-glucosidase, filamentous fungus, and expression cassette containing the modified-type β-glucosidase structural gene sequence are as described above.

### (Vector)

The vector to be used for the transformation method of the present invention contains the above-described expression cassette. Further, a selection marker may preferably be contained in the vector. For example, a vector containing an auxotrophic complementation marker proper for the auxotrophy of the host is preferably used.

Further, the vector of the present invention preferably contains a secretion signal gene capable of functioning in yeast of the genus *Schizosaccharomyces.* The secretion signal gene is located at the 5' end side of the structural gene sequence encoding β-glucosidase. A heterologous protein to which the secretion signal is attached at its N-terminal is expressed from a heterologous protein structural gene to which the secretion signal gene is bound at its 5' end side. The secretion signal is removed from the protein in the endoplasmic reticulum and the Golgi apparatus, etc. of the host cell, and then, the heterologous protein detached from the secretion signal is secreted out of the host cell. The secretion signal gene (and the secretion signal) should be capable of functioning in yeast of the genus *Schizosaccharomyces.* As the secretion signal gene capable of functioning in yeast of the genus *Schizosaccharomyces,* the secretion signal genes described in WO1996/23890 may be used.

The vector to be used for the transformation method of the present invention is a vector having a circular DNA structure or a linear DNA structure. In the case of preparing a transformant in which the above-described expression cassette is maintained in the cells of yeast of the genus *Schizosaccharomyces* as an extrachromosomal gene, the vector is preferably a plasmid which contains a sequence required for replication in yeast of the genus *Schizosaccharomyces,* i.e. Autonomously Replicating Sequence (ARS).

On the other hand, in the case of preparing a transformant in which the above-described expression cassette is integrated into the chromosomes of yeast of the genus *Schizosaccharomyces,* the vector is preferably introduced into the host cells in the form of a linear DNA structure.

A transformant in which the expression cassette is integrated into the chromosomes of yeast of the genus *Schizosaccharomyces* is preferred from the viewpoint of subculture passage of the transformant. The integrated expression cassettes are unlikely to be lost, whereby the passage stability becomes quite high. That is, the transformant in which the expression cassette is integrated into its chromosomes can produce a heterologous protein with more stable productivity.

Here, the vector for preparing the transformant in which the expression cassette is integrated into the chromosomes of yeast of the genus *Schizosaccharomyces* will be described.

The integration of the expression cassette into the chromosomes is preferably carried out by homologous recombination since it is possible to integrate the expression cassette into any position of the chromosomes by homologous recombination.

When introducing the vector into the host cells in the form of a linear DNA structure, in the case of using a vector having a circular DNA structure like a usual plasmid DNA, it is preferred that the vector is cut open to a linear form by a restriction enzyme before introduction to yeast of the genus *Schizosaccharomyces* cells. In this case, the vector having a circular DNA structure is cut open at a position within the recombination region. The resulting vector has parts of the recombination regions exist at both ends and is integrated entirely into the target site in the chromosomes by homologous recombination.

The vector may be constructed by other methods without cutting a vector having a circular DNA structure, for example, by enzymatic amplification using PCR or a chemical synthesis, so long as a linear DNA structure having parts of the recombination region at both ends can be obtained.

To construct the vector of the present invention, a plasmid derived from *E. coli* such as pBR322, pBR325, pUC118, pUC119, pUC18, pUC19 or the like may suitably be used. A vector constructed by using a plasmid derived from *E. coli* usually has the replication origin region called "ori" which is necessary for replication in *E. coli.* Further, even in a case where a plasmid derived from *E. coli* like those mentioned above is not used, when *E. coli* is used for construction and amplification of the vector of the present invention, the "ori" is utilized to obtain a vector containing "ori".

It is preferred that the replication origin region called "ori" required for replication in *E. coli* is removed from the vector for homologous recombination. Accordingly, it is possible to improve the integration efficiency at the time of integrating the above-described vector into a chromosome (refer to JP-A-2000-262284).

The method for constructing the vector in which the replication origin region is removed is not particularly limited, but is preferably the method disclosed in JP-A-2000-262284. That is, it is preferable to preliminarily construct a precursor vector carrying the replication origin region at a position to be cut within the recombination region so that the replication origin region will be cut-off from the vector at the time of preparing a linear DNA structure. Thus, a vector in which the replication origin region is removed is obtained easily.

Further, it may be a method wherein a precursor vector containing an expression cassette and a recombination region is constructed by using the vectors and their construction methods disclosed in JP-A-5-15380, JP-A-7-163373, WO96/23890, JP-A-10-234375, and then the replication origin region is removed from the precursor vector by using a usual genetic engineering method to obtain a vector to be used for homologous recombination.

The number of copies of the expression cassette in the vector may be only one, or two or more. The vector to be used for the transformation method of the present invention preferably contains from 1 to 8, particularly preferably from 2 to 4 copies of the expression cassette.

When the number of copies of the expression cassette in the vector is at least 2, it becomes easier to increase the number of copies of the expression cassette integrated into the chromosomes of yeast of the genus *Schizosaccharomyces* and expression of a heterologous protein. For example, when the number of copies of the expression cassette in the vector is at least 2, a plurality of the expression cassettes may be integrated sequentially in the same position of the chromosomes of yeast of the genus *Schizosaccharomyces.* Further, when the number of copies of the expression cassette in the vector is at most 8, reduction in the efficiency of vector integration via homologous recombination, which happens in a case where the vector size is too large, is likely to be suppressed. When the number of copies of the expression cassette is at most 4, the vector integration efficiency can be improved further.

When the number of the after-mentioned target site in the chromosomes is large, many copies of the expression cassette can be integrated even if the vector has one copy of the expression cassette. Further, two or more copies of the expression cassette may be integrated into the same position of the chromosomes of yeast of the genus *Schizosaccharomyces.*

Further, by setting the number of copies of the expression cassette in the vector to one, and the number of the after-mentioned target site in the chromosomes to one, the amount of modified-type β-glucosidase expressed in each transformant can be adjusted to a uniform level. For example, when evaluating the effect of the introduction of various mutations on the glucosidase activity, by adjusting the amount of modified-type β-glucosidase expressed in each transformant to a uniform level, and then comparing the glucosidase activity on a dry cell weight basis of each transformant (or on a supernatant weight basis in a case where the modified-type β-glucosidase is secreted out of the cells), the glucose activities of modified-type β-glucosidase produced in various transformants can be compared for evaluation.

The recombination region of the vector is a region having a nucleotide sequence which can induce homologous recombination with a target site in the chromosomes of yeast of the genus *Schizosaccharomyces* at which homologous recombination is to be achieved. Further, the target site is a site to become a target for integration of an expression cassette in the chromosomes of yeast of the genus *Schizosaccharomyces.* The target site can be designed freely by letting the recombination region of the vector have a nucleotide sequence which induces homologous recombination with the target site.

To induce homologous recombination, the recombination region is required to have a nucleotide sequence with a homology of at least 70% with the target site. Further, the nucleotide sequence homology between the recombination region and the target site is preferably at least 90%, more preferably at least 95%, in view of increasing the efficiency of homologous recombination. By using a vector containing such a recombination region, the expression cassette is integrated into the target site by homologous recombination.

The length of the recombination region is preferably from 20 to 2,000 bp. When the length of the recombination region is at least 20 bp, homologous recombination is likely to be induced. Further, when the length of the recombination region is at most 2,000 bp, reduction in the homologous recombination efficiency due to too large vector size is likely to be prevented. The length of the recombination region is preferably at least 100 bp, more preferably at least 200 bp. Further, the length of the recombination region is preferably at most 800 bp, more preferably at most 400 bp.

### (Target site in host)

When integrating only one copy of the expression cassette into the chromosomes of the host, the target site in all chromosomes of yeast of the genus *Schizosaccharomyces* for integration of the expression cassette is preferably a target site which located at a position where the integration does not lead to lost or inactivation of an essential gene. When the number of the target site is one, the number of copies of the expression cassette integrated into the chromosomes is fixed and then the comparison among various mutant strains becomes easier, whereby the selectivity of the modified-type β-glucosidase increases.

The above-mentioned essential gene is a gene whose lost or inactivation leads to inviability, i.e. a gene essential for growth of the transformant of the present invention.

When integrating a plurality of the expression cassettes into the chromosomes of the host, the target site for integration of the expression cassette is preferably a plurality of target sites in all chromosomes of yeast of the genus *Schizosaccharomyces,* which satisfies either one of conditions (1) two or more target sites exist in different chromosomes or (2) two or more target sites exists at a plurality of positions in the same chromosome separated by at least one essential gene, or both of (1) and (2). These two or more target sites have substantially the same nucleotide sequence. Here, "substantially the same nucleotide sequence" means that there is at least 90% nucleotide sequence homology between target sites. The nucleotide sequence homology between target sites is preferably at least 95%, more preferably at least 99%.

When the transformant loses the introduced expression cassettes which are separated by the essential gene, the essential gene is also lost, whereby the transformant cannot grow. Accordingly, during cultivation, the transformant which lost the expression cassettes may not grow along with the transformant retaining the expression cassettes, whereby the heterologous protein production efficiency is unlikely to be reduced. Because the growth rate of the transformant which lost expression cassettes has a higher growth rate in general, it is preferred to introduce the expression cassettes into target sites which satisfy the above condition (2).

As described above, since the target sites are dispersed in the chromosomes of yeast of the genus *Schizosaccharomyces,* the expression cassettes are integrated into the chromosomes in a dispersed manner, whereby the integrated expression cassettes are unlikely to be lost and their passage stability is quite high. Accordingly, it is possible to produce a heterologous protein stably with high productivity.

Further, by designing the target site so as to have substantially the same nucleotide sequence, even in a case where a plurality of target sites exists in different positions, it becomes possible to easily integrate the vector into the respective target sites by using a single type of vector.

In the transformation method of the present invention, when integrating a plurality of the expression cassettes, the number of target site positions where the vector is to be integrated is preferably at least 5. When the number of target site positions is at least 5, it is easier to increase the number of copies of the expression cassette integrated into the chromosomes, whereby the productivity of a heterologous protein increases further.

Further, the number of target site positions is preferably from 10 to 60. When the number of target site positions is at least 10, it is easier to further increase the number of copies of the expression cassette integrated into the chromosomes, whereby the productivity of a heterologous protein increases further. When the number of target site positions is at most 60, reduction in expression of a heterologous protein due to integration of too many copies of the expression cassette into the chromosomes is likely to be suppressed.

The target sites preferably have a nucleotide sequence which exists in a transposon gene Tf2, since it becomes possible to integrate the expression cassettes into the target sites dispersed at plural positions in the chromosomes of yeast of the genus *Schizosaccharomyces* at a time by using a single type of vector. Tf2 is a transposon gene which has a length (the number of nucleotide) of about 4,900 bp and exists in the three chromosomes of yeast of the genus *Schizosaccharomyces* at 13 positions in total, with a nucleotide sequence homology of 99.7% (refer to Nathan J. Bowen et al, Genome Res. 2003 13: p1984-1997).

However, the target sites are not limited to the above-described ones. Other than the transposon gene Tf2, the target sites may, for example, be sites (such as genes) found at plural positions in the chromosomes, and having a length larger than the length of the recombination region and a substantially identical nucleotide sequence. Further, for example, after formation of the target site by newly introducing a plurality of genes (target sites) having a substantially identical nucleotide sequence and a length larger than the length of the recombination region into the chromosomes, the vector may be introduced into a plurality of target sites.

### (Transformation method)

The yeast of the genus *Schizosaccharomyces* host is transformed by using the above-described vector. As the transformation method, any known transformation method for yeast of the genus *Schizosaccharomyces* may be used. Such a transformation method may, for example, be a conventional method like a lithium acetate method, electroporation method, spheroplast method, glass-beads method, or the like., and a method disclosed in JP-A-2005-198612. Further, a commercially available yeast transformation kit may be used.

After transformation, the resulting transformants are usually subjected to selection. The selection may, for example, be carried out as follows. Several transformants are selected as viable colonies in a broth *via* the above-mentioned auxotrophic marker screening method, the transformants are grown separately in a liquid broth, and transformants highly expressing a heterologous protein are selected by measuring the amount of a heterologous protein expressed in each transformant (or secreted to a supernatant in a case where the heterologous protein is secreted out of the cells). The number of vectors and copies of the expression cassette integrated into the chromosomes can be identified by analyzing the genomes of the selected transformants by pulse-field gel electrophoresis.

### (Cultivation method)

The transformant of the present invention may be cultivated in the same manner as a natural yeast of the genus *Schizosaccharomyces.*

As the culture broth for cultivating the transformant of the present invention, a known culture broth for yeasts may be used so long as it contains carbon sources, nitrogen sources, inorganic salts and the like which yeast of the genus *Schizosaccharomyces* can use, and yeast of the genus *Schizosaccharomyces* can grow in it efficiently. The culture broth may be natural or synthetic.

As the carbon sources, saccharides such as glucose, fructose and sucrose may, for example, be mentioned.

As the nitrogen sources, inorganic acids or inorganic ammonium salts such as ammonia, ammonium chloride, and ammonium acetate, peptone and casamino acid may, for example, be mentioned.

As inorganic salts, magnesium phosphate, magnesium sulfate and sodium chloride may, for example, be mentioned.

Cultivation may be carried out by using a known cultivation method for yeasts such as a shaking cultivation, a stirring cultivation or the like.

The cultivation temperature is preferably from 23 to 37°C. Further, the cultivation time may be set appropriately.

Cultivation may be carried out batch-wise or continuously.

The transformant of the present invention is a yeast of the genus *Schizosaccharomyces* which can which can produce a modified-type β-glucosidase having higher thermostability or higher resistance to glucose inhibition than a wild-type β-glucosidase. The transformant of the present invention can produce the modified-type β-glucosidase, whereby it can utilize cellobiose.

### [Method for producing β-glucosidase]

The modified-type β-glucosidase can be recovered from the cells obtained by cultivating the transformant of the present invention. When the transformant is cultivated to isolate the modified-type β-glucosidase from culture broth or cells, a known protein isolation method may be used. For example, the cells are separated from the culture broth after cultivation, and then the separated cells are disrupted to obtain a cell lysate containing the modified-type β-glucosidase, followed by recovery of the modified-type β-glucosidase from the cell lysate by using a known protein isolation method such as salting-out, column chromatography purification or immunoprecipitation.

Further, in the case of using a transformant in which an expression cassette containing a structural gene sequence comprising a region encoding the modified-type β-glucosidase and a region encoding a secretion signal attached at the 5' end of the region encoding the modified-type β-glucosidase is introduced, the modified-type β-glucosidase produced by the transformant is secreted out of the cells. Therefore, it is possible to recover the modified-type β-glucosidase from a culture supernatant by using a known protein isolation method. The transformant may be cultivated continuously to produce the modified-type β-glucosidase by repeating recovery of a culture supernatant containing the modified-type β-glucosidase from the culture broth by means of centrifugation or the like after a certain period of cultivation, and recultivation of the remained cells after supplementing them with a culture broth.

### [Cellulose degradation method]

In the cellulose degradation method of the present invention, the modified-type β-glucosidase recovered from the cells or the culture broth obtained by cultivating the above-described transformant of yeast of the genus *Schizosaccharomyces* is used.

The form of cellulose to be degraded is not particularly limited, and may be purified cellulose or cellulose contained in a biomass such as a wood, rice straw, rice husk and weed.

As the specific cellulose degradation method, a method wherein the transformant is cultivated to isolate or purify a modified-type β-glucosidase from the culture broth or the cells, and then the isolated or purified modified-type β-glucosidase is cultivated along with a biomass containing cellulose, an endoglucanase, and a cellobiohydrolase may, for example, be mentioned. In the case of decomposing cellulose by using the isolated and purified modified-type β-glucosidase produced by the transformant, the reaction conditions may be known reaction conditions for β-glucosidase. The suitable pH for the modified-type β-glucosidase in the degradation reaction solution is from 3.0 to 8.0, and the suitable reaction temperature is from 20 to 65°C.

The above-mentioned culture supernatant or cell lysate may be directly contacted with cellulose without isolating the modified-type β-glucosidase from the culture supernatant or cell lysate containing the modified-type β-glucosidase. For example, in the case of using a transformant in which an expression cassette containing a region encoding the modified-type β-glucosidase and a region encoding a secretion signal at the 5' end of the region encoding the modified-type β-glucosidase is introduced, the transformant may be cultivated in a culture broth containing cellulose thereby to degrade the cellulose contained in the culture broth by the modified-type β-glucosidase secreted into the culture broth. As the specific cellulose degradation method, a method wherein the transformant is cultivated in the presence of the above-mentioned biomass, a method wherein the transformant is cultivated and then mixed with the above-mentioned biomass for degradation, etc. may, for example, be mentioned. In such a case, a general cultivation condition for yeast of the genus *Schizosaccharomyces* may be used as the cultivation condition. The suitable pH of the culture broth is from 3.0 to 8.0, and the suitable cultivation temperature is from 23 to 37°C.

### EXAMPLES

Now, the present invention will be described in further detail with reference to Examples and Comparative Examples. However, it should be understood that the present invention is by no means thereby restricted.

### [Example 1]

### <Preparation of a Schizosaccharomyces pombe transformant which can produce AaBGL1>

A gene sequence was designed based on the peptide sequence of AaBGL1 represented by SEQ ID NO: 1, by replacing the codons with codons highly expressed in *Schizosaccharomyces pombe* (refer to SEQ ID NO: 2. hereinafter referred to as AaBGL1 gene). The recognition sequences for KpnI and BspHI were added upstream of the initiation codon. The recognition sequences for XbaI and SacI were added downstream of the stop codon. A plasmid containing these sequences (synthesized by Geneart AG, Regensburg, Germany) was digested with restriction enzymes BspHI and XbaI.

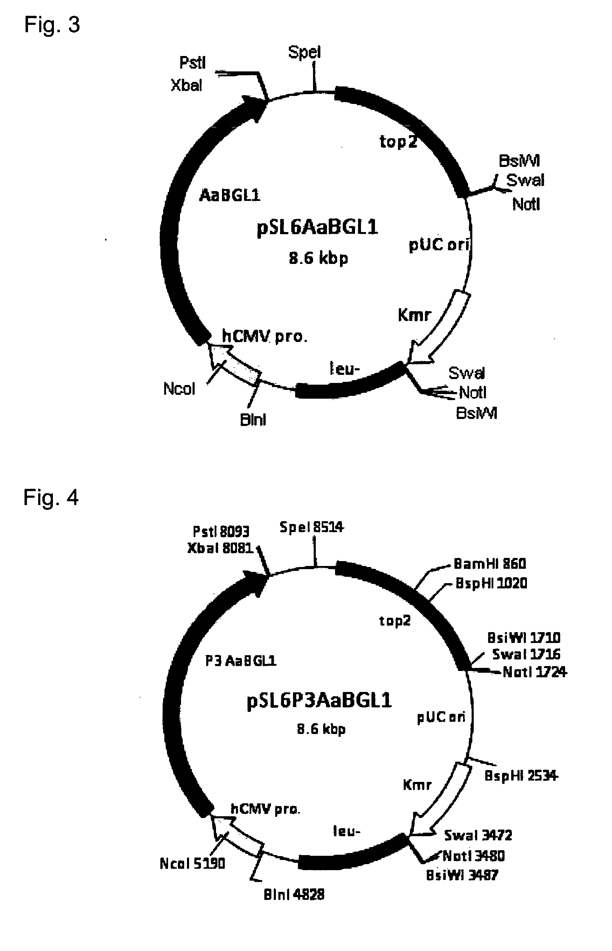

On the other hand, separately therefrom, pSL6lacZ was digested with restriction enzymes Aarl and XbaI, and then treated with an alkaline phosphatase. Thereafter, gel electrophoresis was carried out on an agarose gel to isolate the digested fragment of vector pSL6 and the digested fragment of AaBGL1 gene from the agarose gel, and then these fragments were ligated to each other. The ligated product was introduced into *E. coli* DH5α (Takara Bio, Inc.) to obtain a transformant. From the obtained transformant, a vector was prepared to obtain expression vector pSL6AaBGL1 (Fig. 3, refer to SEQ ID NO: 3). The obtained expression vector was confirmed to be a desired vector by restriction enzyme mapping.

Further, to prepare P3AaBGL1 in which secretion signal P3 is bound at the N-terminal of AaBGL1 having a 19 amino acid deletion at its N-terminal (refer to SEQ ID NO: 4), a fragment of AaBGL1 gene was amplified by PCR method with In-fusion primers and pSL6AaBGL1 as template. On the other hand, pSL6P3lacZ was digested with restriction enzymes AfIII and XbaI. The digested fragments and the PCR-amplified product of the AaBGL1 gene fragment were circularized by In-fusion method, and then introduced into *E. coli* DH5α (Takara Bio, Inc.) to obtain a transformant. From the obtained transformant, a vector was prepared to obtain expression vector pSL6P3AaBGL1 (Fig. 4, refer to SEQ ID NO: 5). The obtained expression vector was confirmed to be a desired vector by restriction enzyme mapping and partial nucleotide sequencing.

As the host cell, a leucine-auxotrophic strain of *Schizosaccharomyces pombe* (genotype: h-, leu1-32, provided from professor Yuichi lino, Molecular Genetics Research Laboratory, Graduate School of Science, The University of Tokyo) (ATCC38399) was cultivated in YES medium (0.5% of yeast extract, 3% of glucose and 0.1 mg/ml of SP supplements) until 0.6 x 10⁷ cells/ml. The cells were collected and washed, and then suspended by 0.1 M lithium acetate solution (pH 5.0) to 1.0 x 10⁸ cells/ml. Thereafter, to 100 µl of the suspension, 1 µg of the above-mentioned vector pSL6P3AaBGL1 digested by restriction enzyme Notl was added, and then 290 µl of a 50 % (w/v) polyethylene glycol (PEG4000) aqueous solution was added thereto, followed by stirring to cultivate them for 60 minutes at 30°C, 5 minutes at 42°C, and 10 minutes at room temperature, in this order. PEG4000 was removed by centrifugation and then the cells were washed to suspend them in 150 µl of sterile water. The suspension was applied on minimal-agarose medium.

Three days after cultivation, a transformant (AaBGL1 expression strain) was obtained. Thus obtained transformant was designated as ASP3326 strain.

### <Preparation of error-prone PCR fragment>

By using pSL6AaBGL1 as template, a fragment of P3AaBGL1 gene was amplified by error-prone PCR method with the primers of SEQ ID NO: 6 and SEQ ID NO: 7. The error-prone PCR was carried out by using Diversify PCR Random Mutagenesis Kit (manufactured by Clontech Lab., Inc.). On the other hand, separately therefrom, by using pSL6P3AaBGL1 as a template, a fragment of leu1⁺ promoter was amplified by PCR method with the primers of SEQ ID NO: 8 and SEQ ID NO: 9. Further, separately therefrom, by using pSL6P3AaBGL1 as a template, a fragment of terminator top2 was amplified by PCR method with the primers of SEQ ID NO: 10 and SEQ ID NO: 11. For the PCR method, PrimeSTAR Max DNA polymerase (manufactured by Takara Bio Inc.) was used.

To a reaction mixture comprised of these three types of fragments, restriction enzyme DpnI was added to digest the templates, and then the fragments were purified by using SUPREC-PCR (manufactured by Takara Bio Inc.) for primer substitution. By using 50 ng of the purified three types of fragments, a mutant gene-integrated fragment comprising leu1⁺ promoter, mutant-type P3AaBGL1, and terminatortop2 (leu1⁺ promoter-mutant-type P3AaBGL1-terminator top2) was amplified by PCR method with the primers of SEQ ID NO: 12 and SEQ ID NO: 13. Thereafter, a column purification was carried out by using SUPREC-PCR.

### <Preparation of transformant>

As the host cell, a leucine-auxotrophic strain of *Schizosaccharomyces pombe* (genotype: h-, leu1-32, provided from professor Yuichi lino, Molecular Genetics Research Laboratory, Graduate School of Science, The University of Tokyo) (ATCC38399) was cultivated in YES medium (0.5% of yeast extract, 3% of glucose and 0.1 mg/ml of SP supplements) until 0.6 x 10⁷ cells/ml. The cells were collected and washed, and then suspended by 0.1 M lithium acetate solution (pH 5.0) to 1.0 x 10⁸ cells/ml. Thereafter, to 100 µl of the suspension, 1 µg of the leu1⁺ promoter-P3AaBGL1-terminator top2 fragment was added, and then 290 µl of a 50 % (w/v) polyethylene glycol (PEG4000) aqueous solution was added thereto, followed by stirring to cultivate them for 60 minutes at 30°C, 5 minutes at 42°C, and 10 minutes at room temperature, in this order. PEG4000 was removed by centrifugation and then the cells were washed to suspend them in 150 µl of sterile water. The suspension was applied on minimal-agarose medium. The composition of the minimal-agarose medium is shown in Tables 1 to 6.

By limiting the carbon sources of the minimal-agarose medium to 0.1 % glucose and 2% cellobiose, it is possible to select an active type P3AaBGL1 mutant strain based on the formation of large colonies, since only the active type P3AaBGL1 mutant strain can utilize cellobiose as a carbon source.

Five days after cultivation, a transformant (P3AaBGL1 mutant strain) was obtained. The obtained transformant was designated as P3AaBGL1 mutant strain library.

**TABLE 1**

| 10xMA composition | |
|---|---|
| Potassium hydrogen phthalate | 3 g |
| Na₂HPO₄ | 2.2 g |
| NH₄Cl | 5 g |
| Water | Up to 1000 ml |

**TABLE 2**

| Salt stock solution composition | |
|---|---|
| MgCl₂ 6H₂O | 53.3 g |
| CaCl₂ 2H₂O | 0.735 g |
| KCI | 50 g |
| Na₂SO₄ | 2 g |
| Water | Up to 1000 ml |

**TABLE 3**

| Mineral stock solution composition | |
|---|---|
| H₃BO₃ | 500 mg |
| MnSO₄ 5H₂O | 530 mg |
| ZnSO₄ 7H₂ | 400 mg |
| FeCl₃ 6H₂O | 200 mg |
| Na₂MoO₄ 2H₂O | 200 mg |
| KI | 100 mg |
| CuSO₄ 5H₂O | 40 mg |
| Potassium dihydrogen citrate | 1000 mg |
| Water | Up to 1000 ml |

**TABLE 4**

| Vitamin stock solution composition | |
|---|---|
| Pantothenic acid Ca | 0.01 g |
| Nicotinic acid | 0.10 g |
| Inositol | 0.10 g |
| Water | Up to 10 ml |

**TABLE 5**

| Biotin stock solution composition | |
|---|---|
| Biotin | 0.001 g |
| Water | Up to 10 ml |

**Table 6**

| Minimal-agarose medium composition | |
|---|---|
| 10xMA | 100 ml |
| Salt stock solution | 20 ml |
| Mineral stock solution | 1 ml |
| Vitamin stock solution | 1 ml |
| Biotin stock solution | 1 ml |
| Glucose * | 20 g |
| Aqarose | 30 g |
| Water | 880ml |

### <Studies on carbon sources for minimal-agarose medium>

By limiting the carbon sources (indicated by * in Table 6) of the minimal-agarose medium to 0.1% glucose and 2% cellobiose, only an active type P3AaBGL1 mutant strain (transformant which produces an AaBGL1 mutant having a glucosidase activity) can utilize cellobiose as a carbon source. Accordingly, as described below, it becomes possible to select an active P3AaBGL1 mutant strain based on the formation of large colonies, by cultivating transformants in the minimal-agarose medium.

The colony formation and the BGL activity of a P3AaBGL1 mutant strain were compared with the case where 2% glucose was used as a carbon source. The BGL activity of a P3AaBGL1 mutant strain formed by using a minimal-agarose medium having glucose as a carbon source was measured in accordance with the following activity measurement method, and a result, the percentage of mutant strains having a BGL activity were found to be 26.5%, based on the total colonies. In comparison to this result, 70.5% of P3AaBGL1 mutant strains, based on the total P3AaBGL1 mutant strains formed in the minimal-agarose medium having 0.1 % glucose and 2% cellobiose as carbon sources, were found to form small colonies. The BGL1 activities of the remaining P3AaBGL1 mutant strains which formed relatively large colonies were measured in accordance with the following activity measurement method, and as a result, the percentage of AaBGL1 mutant strains having a BGL activity was found to be 83.9%. That is, by selectively recovering P3AaBGL1 mutant strains which formed large colonies in the minimal-agarose medium having 0.1% glucose and 2% cellobiose as carbon sources, it was possible to exclude P3AaBGL1 mutant strains which had lost a BGL activity. Further, in the case where the carbon source was only 2% cellobiose, the recovery efficiency of transformants were significantly decreased, whereby recovery of large number of mutant strains was difficult. This is presumably because the transformants cannot utilize cellobiose due to lack of carbon sources required for expressing mutant-type P3AaBGL1 retaining the activity.

### <Screening from P3AaBGL1 mutant strain library>

A mutant strain which formed a large colony was selected from P3AaBGL1 mutant strain library. Then, thus selected strain and P3ASP3326 strain as the original strain were cultivated in 150 µl/well (96-well plate) of YES medium at 30°C for two days. 50 µl of each culture supernatant was diluted by 150 µl of 50 mM acetate buffer (pH 5.0) to prepare a diluted enzyme sample. Then, the enzymatic activity of the sample was measured in accordance with the following method.

### (Activity measurement method)

To 10 µl of 20 mM p-nitrophenyl-β-D-glucoside (hereinafter abbreviated as pNPG), 10 µl of 1 M sodium acetate buffer solution (pH 5.0) and 130 µl of water were added, and then 50 µl of the diluted enzyme sample was introduced for reacting them at 37°C for 10 minutes. 100 µl of the reaction mixture was mixed with 100 µl of 2% sodium carbonate solution to terminate reaction, and then the amount of free p-nitrophenol was colorimetrically measured at a wavelength of 450 nm.

The amount of enzyme that produces 1 µmol of p-nitrophenol per minute was defined as 1 U.

### (Glucose inhibition measurement)

To 10 µl of 20 mM pNPG, 10 µl of 1 M sodium acetate buffer solution (pH 5.0) and from 4 to 10 µl of 1 M glucose (final concentration of from 20 to 50 mM) were added, followed by addition of water up to the total volume of 150 µl. Thereafter, 50 µl of the diluted enzyme sample was introduced for reacting them at 37°C for 10 minutes. As a control, a glucose-free reaction mixture was used, and then the reaction was carried out at 37°C for 10 minutes, in the same manner. 100 µl of each reaction mixture was mixed with 100 µl of 2% sodium carbonate solution to terminate reaction, and then the amount of liberated p-nitrophenol was colorimetrically measured at a wavelength of 450 nm.

The ratio (%) of the colorimetric value of each reaction mixture to the colorimetric value of the control sample (glucose-free sample) was defined as the residual activity after glucose inhibition. The residual activities of each of the mutants were compared with the results obtained by using ASP3326 strain, thereby to select a mutant strain showed changes in resistance to glucose inhibition.

### (Thermostability measurement)

To 10 µl of 20 mM pNPG, 10 µl of 1 M sodium acetate buffer solution (pH 5.0) and 130 µl of water were added, and then 50 µl of a diluted enzyme sample preliminarily heat-treated at from 65 to 70°C for 10 minutes was introduced for reacting them at 37°C for 10 minutes. As a control, a diluted enzyme sample which was not subjected to the heat treatment was used, and then the reaction was carried out at 37°C for 10 minutes, in the same manner. 100 µl of each reaction mixture was mixed with 100 µl of 2% sodium carbonate solution to terminate reaction, and then the amount of liberated p-nitrophenol was colorimetrically measured at a wavelength of 450 nm.

The ratio (%) of the colorimetric value of each reaction mixture to the colorimetric value of the control sample (untreated sample) was defined as the residual activity after heat treatment. The residual activities of each of the mutants were compared with the results obtained by using ASP3326 strain, thereby to select a mutant strain showed improved thermostability.

### <Cultivation of recovered mutant strain>

The P3AaBGL1 mutant strain obtained as above was cultivated in YES medium at 30°C for one day. 100 µl of the culture broth was inoculated on 5 ml of YPD medium (1 % of yeast extract, 2% of peptone, and 2% of glucose), followed by cultivation at 30°C for two days. Thereafter, the culture broth was centrifuged to obtain a culture supernatant.

### <Evaluation of recovered mutant strain>

By using the above-obtained culture supernatant of the P3AaBGL1 mutant strain, a diluted enzyme sample was prepared, and then the enzymatic activity of the sample was measured in accordance with the above-described activity measurement method. Further, the glucose inhibition at a final glucose concentration of from 0.1 to 200 mM and the thermostability at a warming temperature of from 50 to 75°C were measured for evaluation.

### <Recovery of modified-type P3AaBGL1 gene>

From the P3AaBGL1 mutant strains obtained as above, a P3AaBGL1 mutant strain showed changes in the resistance to glucose inhibition and the thermostability of glucosidase, as compared to those of ASP3326 strain, was selected. 100 µl of the culture broth of the selected P3AaBGL1 mutant strain was centrifuged to collect cells. The collected cells were treated with Zymolyase, and thus treated cells were used as templates for amplifying a gene-integrated fragment by PCR method using the primers of SEQ ID NO: 14 and SEQ ID NO: 15, and PrimeSTAR Max DNA polymerase. Further, by using thus obtained fragment as a template, a fragment of P3AaBGL1 mutant gene was amplified by PCR method with In-fusion primers (the primers of SEQ ID NO: 16 and SEQ ID NO: 17). On the other hand, pSL6P3lacZ was digested with restriction enzymes Aflll and Xbal. The digested fragments and the PCR-amplified product of the P3AaBGL1 mutant gene fragment were circularized by In-fusion method, and then introduced into *E. coli* DH5α to obtain a transformant. From the obtained transformant, a vector was prepared to obtain expression vector pSL6P3AaBGL1 mutXX (XX indicates strain name).

The nucleotide sequences of each P3AaBGL1 gene in pSL6P3AaBGL1 mutXX vector and the P3AaBGL1 mutant gene fragment were confirmed to identify the mutation site.

The above-described operation was repeated independently four times, and as a result, seven P3AaBGL1 mutant strains having P3AaBGL1 in which mutations affecting the resistance to glucose inhibition and the thermostability of the glucosidase expressed from the modified-type P3AaBGL1 gene were introduced, were obtained. With regard to modified-type P3AaBGL1 and P3AaBGL1 produced by the respective P3AaBGL1 mutant strains, the types of mutation introduced into P3AaBGL1, the glucosidase activity, the residual glucosidase activity in a culture broth containing glucose at a concentration of 20 mM or 50 mM (a value relative to the value obtained when the glucose concentration was 0 mM), and the residual glucosidase activity after warming a culture broth for 10 minutes at a warming temperature of 65°C or 67°C (a value relative to the value obtained when the culture broth was not subjected to preliminary heat treatment) were measured, and the results are shown in Table 7. In the Table, "Activity" indicates glucosidase activity and "Silent" indicates the number of silent mutation found in each mutant strain. Further, "w. t." indicates the results of ASP3326 strain. These results suggest that Q142 (glutamine at position 142, corresponding to glutamine at position 126 of AaBGL1), Q156 (glutamine at position 156, corresponding to glutamine at position 140 of AaBGL1), I260 (isoleucine at position 260, corresponding to isoleucine at position 244 of AaBGL1), G298 (glycine at position 298, corresponding to glycine at position 282 of AaBGL1), N458 (asparagine at position 458, corresponding to asparagine at position 442 of AaBGL1), G468 (glycine at position 468, corresponding to glycine at position 452 of AaBGL1), V519 (valine at position 519, corresponding to valine at position 503 of AaBGL1), and R629 (arginine at position 629, corresponding to arginine at position 613 of AaBGL1) of P3AaBGL1 are involved in the resistance to glucose inhibition or the thermostability.

**TABLE 7**

| | Activity [mU/ml] | Residual activity [%] | | | | Mutation site | | | | | | | | | | | | Silent |
|---|---|---|---|---|---|---|---|---|---|---|---|---|---|---|---|---|---|---|
| | | Glc 20mM | Glc 50mM | Temp 65°C | Temp 67°C | I19 | Q142 | Q156 | I260 | G298 | N458 | G468 | V519 | R629 | Q681 | T693 | D744 | |
| w. t. | 680 | 40 | 22 | 61 | 44 | | | | | | | | | | | | | |
| 6H02 | 446 | 22 | 12 | 46 | 33 | | | | | S | | | | | H | | N | 1 |
| 6H05 | 525 | 41 | 23 | 73 | 59 | | | | | | | | A | | | | | 4 |
| 9H03 | 647 | 65 | 43 | 38 | 23 | | | L | F | F | | | | | | | | 4 |
| 9H08 | 358 | 45 | 26 | 72 | 65 | | | | | | | | A | L | | | | 0 |
| 11F10 | 322 | 84 | 72 | 7 | 2 | | | L | F | F | | S | | | | | | 3 |
| 18A08 | 337 | 44 | 26 | 82 | 75 | | | | | | S | | A | L | | A | | 1 |
| 18A12 | 445 | 44 | 26 | 82 | 77 | N | L | | | | | | A | L | | | | 3 |
| 22A12 | 501 | 43 | 25 | 83 | 75 | | L | | | | S | | A | L | | | | 1 |

### [Example 2]

### <Preparation of G298X Site-Saturation Mutagenesis fragment>

Specific amino acid substitution of G298 was carried out with degenerate primers. By using pSL6P3AaBGL1 as a template, a leu1⁺-G298X fragment was amplified by PCR method with the primers of SEQ ID NO: 8 and SEQ ID NO: 18. On the other hand, a G298X-top2 fragment was amplified by PCR method with the primers of SEQ ID NO: 19 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-G298X-top2 fragment was amplified by PCR method with the primers of SEQ ID NO: 12 and SEQ ID NO: 13. Thereafter, a column purification was carried out by using SUPREC-PCR.

### <Recovery of mutant strain obtained by Site-Saturation Mutagenesis>

In the same manner as in Example 1, except that the leu1⁺-G298X-top2 fragment was used as a mutant gene-integrated fragment, mutant strains were obtained, and then the glucose inhibition and the thermostability at a warming temperature of from 50 to 75°C of the culture supernatants of the respective mutant strains were measured. Thereafter, a mutant strain showed changes in any of the activities as compared to ASP3326 strain was selected, and then the nucleotide sequence of P3AaBGL1 gene integrated into the chromosomes of the selected mutant strain was confirmed, thereby to identify an amino acid at position 298. As a result, in a P3AaBGL1 mutant strain showed changes in the resistance to glucose inhibition or the thermostability as compared to ASP3326 strain, the amino acid at position 298 was identified as serine, phenylalanine or proline.

### [Example 3]

### <Preparation of V519X Site-Saturation Mutagenesis fragment>

Specific amino acid substitution of V519 was carried out with degenerate primers. By using pSL6P3AaBGL1 as a template, a leu1⁺-V519X fragment was amplified by PCR method with the primers of SEQ ID NO: 8 and SEQ ID NO: 20. On the other hand, a V519X-top2 fragment was amplified by PCR method with the primers of SEQ ID NO: 21 and SEQ ID NO: 11.

To a reaction mixture comprised of these two types of fragments, restriction enzyme Dpnl was added to digest the templates, and then the fragments were purified by using SUPREC-PCR for primer substitution. By using 50 ng of the purified two types of fragments, a leu1⁺-V519X-top2 fragment was amplified by PCR method with the primers of SEQ ID NO: 12 and SEQ ID NO: 13. Thereafter, a column purification was carried out by using SUPREC-PCR.

### <Recovery of mutant strain obtained by Site-Saturation Mutagenesis>

In the same manner as in Example 1, except that the leu1⁺-V519X-top2 fragment was used as a mutant gene-integrated fragment, mutant strains were obtained, and then the glucose inhibition and the thermostability at a warming temperature of from 50 to 75°C of the culture supernatants of the respective mutant strains were measured. Thereafter, a mutant strain showed changes in any of the activities as compared to ASP3326 strain was selected, and then the nucleotide sequence of P3AaBGL1 gene integrated into the chromosomes of the selected mutant strain was confirmed, thereby to identify an amino acid at position 519. As a result, in a P3AaBGL1 mutant strain showed changes in the resistance to glucose inhibition or the thermostability as compared to ASP3326 strain, the amino acid at position 519 was identified as alanine, glycine or cysteine.

### [Example 4]

### <Preparation of Q142X Site-Saturation Mutagenesis fragment>

Specific amino acid substitution of Q142 was carried out with degenerate primers. By using pSL6P3AaBGL1 as a template, a leu1⁺-Q142X fragment was amplified by PCR method with the primers of SEQ ID NO: 8 and SEQ ID NO: 22. On the other hand, a Q142X-top2 fragment was amplified by PCR method with the primers of SEQ ID NO: 23 and SEQ ID NO: 11.

To a reaction mixture comprised of these two types of fragments, restriction enzyme Dpnl was added to digest the templates, and then the fragments were purified by using SUPREC-PCR for primer substitution. By using 50 ng of the purified two types of fragments, a leu1⁺-Q142X-top2 fragment was amplified by PCR method with the primers of SEQ ID NO: 12 and SEQ ID NO: 13. Thereafter, a column purification was carried out by using SUPREC-PCR.

### <Recovery of mutant strain obtained by Site-Saturation Mutagenesis>

In the same manner as in Example 1, except that the leu1⁺-Q142X-top2 fragment was used as a mutant gene-integrated fragment, mutant strains were obtained, and then the glucose inhibition and the thermostability at a warming temperature of from 50 to 75°C of the culture supernatants of the respective mutant strains were measured. Thereafter, a mutant strain showed changes in any of the activities as compared to ASP3326 strain was selected, and then the nucleotide sequence of P3AaBGL1 gene integrated into the chromosomes of the selected mutant strain was confirmed, thereby to identify an amino acid at position 142. As a result, in a P3AaBGL1 mutant strain showed changes in the resistance to glucose inhibition or the thermostability as compared to ASP3326 strain, the amino acid at position 412 was identified as asparagine or arginine.

### [Example 5]

### <Preparation of G468X Site-Saturation Mutagenesis fragment>

Specific amino acid substitution of G468 was carried out with degenerate primers. By using pSL6P3AaBGL1 as a template, a leu1⁺-G468X fragment was amplified by PCR method with the primers of SEQ ID NO: 8 and SEQ ID NO: 24. On the other hand, a G468X-top2 fragment was amplified by PCR method with the primers of SEQ ID NO: 25 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-G468X-top2 fragment was amplified by PCR method with the primers of SEQ ID NO: 12 and SEQ ID NO: 13. Thereafter, a column purification was carried out by using SUPREC-PCR.

### <Recovery of mutant strain obtained by Site-Saturation Mutagenesis>

In the same manner as in Example 1, except that the leul ⁺-G468X-top2 fragment was used as a mutant gene-integrated fragment, mutant strains were obtained, and then the glucose inhibition and the thermostability at a warming temperature of from 50 to 75°C of the culture supernatants of the respective mutant strains were measured. Thereafter, a mutant strain showed changes in any of the activities as compared to ASP3326 strain was selected, and then the nucleotide sequence of P3AaBGL1 gene integrated into the chromosomes of the selected mutant strain was confirmed, thereby to identify an amino acid at position 468. As a result, in a P3AaBGL1 mutant strain showed changes in the resistance to glucose inhibition or the thermostability as compared to ASP3326 strain, the amino acid at position 468 was identified as serine.

The types of mutation introduced into P3AaBGL1, the glucosidase activity, the residual glucosidase activity in a culture broth containing glucose at a concentration of 20 mM or 50 mM (a relative value), and the residual glucosidase activity after warming a culture broth for 10 minutes at a warming temperature of 65°C or 67°C (a relative value) of the respective mutant strains obtained in Examples 2 to 5 are shown in Table 8.

**TABLE 8**

| | Activity [mU/ml] | Residual activity [%] | | | | Mutation site | | | | Silent |
|---|---|---|---|---|---|---|---|---|---|---|
| | | Glc 20mM | Glc 50mM | Temp 65°C | Temp 67°C | Q142 | G298 | G468 | V519 | |
| w. t. | 680 | 40 | 22 | 61 | 44 | | | | | |
| 7G06 | 362 | 55 | 35 | 39 | 27 | | F | | | 0 |
| 7G09 | 518 | 22 | 11 | 44 | 28 | | S | | | 0 |
| 8A08 | 312 | 63 | 41 | 5 | 6 | | P | | | 0 |
| 7F01 | 209 | 48 | 26 | 67 | 57 | | | | G | 0 |
| 8F08 | 513 | 43 | 23 | 61 | 47 | | | | C | 0 |
| 8F11 | 544 | 42 | 24 | 70 | 58 | | | | A | 0 |
| 32A02 | 582 | 38 | 21 | 70 | 58 | N | | | | 0 |
| 32A03 | 575 | 38 | 20 | 57 | 51 | R | | | | 0 |
| 31C06 | 267 | 75 | 54 | 12 | 2 | | | S | | 0 |

As a result, G298F (substitution of glycine at position 298 to phenylalanine) was found to increase the resistance to glucose inhibition and decrease the thermostability of P3AaBGL1. Further, V519A (substitution of valine at position 519 to alanine) was found to significantly increase the thermostability, while the resistance to glucose inhibition of P3AaBGL1 was not affected thereby. Further, G468S (substitution of glycine at position 468 to serine) was found to increase the resistance to glucose inhibition and decrease the thermostability of P3AaBGL1. Further, it was suggested that the thermostability of P3AaBGL1 can be improved by Q142N (substitution of glutamine at position 142 to asparagine).

### [Example 6]

### <Recombination of mutant genes by StEP method>

By using two or several types of pSL6P3AaBGL1 mutXX vectors (total 1 ng) as templates, fragments of P3AaBGL1 gene were amplified by a StEP method of Zhao et al. (Huimin Zhao, METHODS IN ENZYMOLOGY, Vol. 388, pp. 42-49, 2004) with the primers of SEQ ID NO: 6 and SEQ ID NO: 7, PrimeSTAR HS DNA Polymerase (Takara Bio Inc.) and the following PCR program: 30 cycles of 10 seconds at 98°C, 5 seconds at 55°C, and 20 seconds at 72°C (2.6 kb), followed by 5 cycles of 10 seconds at 98°C, 5 seconds at 55°C, and 3 minutes at 72°C (2.6 kb). On the other hand, separately therefrom, by using pSL6P3AaBGL1 as a template, a fragment of leu1⁺ promoter was amplified by PCR method with the primers of SEQ ID NO: 8 and SEQ ID NO: 9. Further, separately therefrom, by using pSL6P3AaBGL1 as a template, a fragment of terminator top2 was amplified by PCR method with the primers of SEQ ID NO: 10 and SEQ ID NO: 11. For the PCR method, PrimeSTAR Max DNA polymerase (manufactured by Takara Bio Inc.) was used.

To a reaction mixture comprised of these three types of fragments, restriction enzyme DpnI was added to digest the templates, and then the fragments were purified by using SUPREC-PCR (manufactured by Takara Bio Inc.) for primer substitution. By using 50 ng of the purified three types of fragments, a leu1⁺ promoter-P3AaBGL1--terminator top2 fragment was amplified with the primers of SEQ ID NO: 12 and SEQ ID NO: 13. Thereafter, a column purification was carried out by using SUPREC-PCR.

### <Recovery of StEP mutation-recombination strain>

In the same manner as in Example 1, except that the above-prepared fragment was used as a mutant gene-integrated fragment, mutant strains were obtained, and then the glucose inhibition and the thermostability at a warming temperature of from 50 to 75°C of the culture supernatants of the respective mutant strains were measured. Thereafter, a mutant strain showed changes in any of the activities as compared to ASP3326 strain was selected, and then the nucleotide sequence of P3AaBGL1 gene integrated into the chromosomes of the selected mutant strain was confirmed.

The types of mutation introduced into P3AaBGL1, the glucosidase activity, the residual glucosidase activity in a culture broth containing glucose at a concentration of 20 mM or 50 mM, and the residual glucosidase activity after warming a culture broth for 10 minutes at a warming temperature of 65°C or 67°C of the respective mutant strains obtained as above are shown in Table 9.

**TABLE 9**

| | Activity [mU/ml] | Residual activity [%] | | | | Mutation site | | | | | | | | Silent |
|---|---|---|---|---|---|---|---|---|---|---|---|---|---|---|
| | | Glc 20mM | Glc 50mM | Temp 65°C | Temp 67°C | Q142 | Q156 | I260 | G298 | N458 | V519 | R629 | T693 | |
| w. t. | 680 | 40 | 22 | 61 | 44 | | | | | | | | | |
| 13C02 | 357 | 67 | 46 | 63 | 48 | | L | F | F | | A | L | | 0 |
| 22A12 | 501 | 43 | 25 | 83 | 75 | L | | | | S | A | L | | 1 |
| 24A01 | 460 | 43 | 25 | 84 | 74 | L | | | | S | A | L | A | 0 |
| 24A12 | 316 | 66 | 45 | 78 | 67 | | L | F | F | S | A | L | A | 0 |
| 27A10 | 341 | 67 | 45 | 76 | 68 | | L | F | F | S | A | L | | 0 |

### [Example 7]

### <Preparation of Site-Saturation Mutagenesis fragment>

By using pSL6P3AaBGL1 mut27A10 vector as a template, specific amino acid substitutions of Q142N and G468S were carried out with degenerate primers.

When carrying out specific amino acid substitution of Q142N, specifically, a leu1⁺-Q142X fragment was amplified by PCR method with the primers of SEQ ID NO: 8 and SEQ ID NO: 22. On the other hand, a Q142N-top2 fragment was amplified by PCR method with the primers of SEQ ID NO: 23 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-Q142N-top2 fragment was amplified by PCR method with the primers of SEQ ID NO: 12 and SEQ ID NO: 13. Thereafter, a column purification was carried out by using SUPREC-PCR.

Further, when carrying out specific amino acid substitution of G468S, a leu1⁺-Q142X fragment was amplified by PCR method with the primers of SEQ ID NO: 7 and SEQ ID NO: 24. On the other hand, a G468S-top2 fragment was amplified by PCR method with the primers of SEQ ID NO: 25 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-G468S-top2 fragment was amplified by PCR method with the primers of SEQ ID NO: 12 and SEQ ID NO: 13. Thereafter, a column purification was carried out by using SUPREC-PCR.

### <Recovery of mutant strain obtained by Site-Saturation Mutagenesis>

In the same manner as in Example 1, except that each of the above-prepared fragments was used as a mutant gene-integrated fragment, mutant strains were obtained, and then the glucose inhibition and the thermostability at a warming temperature of from 50 to 75°C of the culture supernatants of the respective mutant strains were measured.

The types of mutation introduced into P3AaBGL1, the glucosidase activity, the residual glucosidase activity in a culture broth containing glucose at a concentration of 20 mM or 50 mM, and the residual glucosidase activity after warming a culture broth for 10 minutes at a warming temperature of 65°C or 67°C of the respective mutant strains obtained as above are shown in Table 10.

**TABLE 10**

| | Activity [mU/ml] | Residual activity [%] | | | | Mutation site | | | | | | | | Silent |
|---|---|---|---|---|---|---|---|---|---|---|---|---|---|---|
| | | Glc 20mM | Glc 50mM | Temp 65°C | Temp 67°C | Q142 | Q156 | I260 | G298 | N458 | G468 | V519 | R629 | |
| w. t. | 680 | 40 | 22 | 61 | 44 | | | | | | | | | |
| 28C02 | 350 | 66 | 43 | 77 | 70 | N | L | F | F | S | | A | L | 0 |
| 29F09 | 215 | 87 | 69 | 38 | 8 | | L | F | F | S | S | A | L | 0 |
| mutBGL1 | 235 | | 65 | | 11 | N | L | F | F | S | S | A | L | 0 |

### [Example 8]

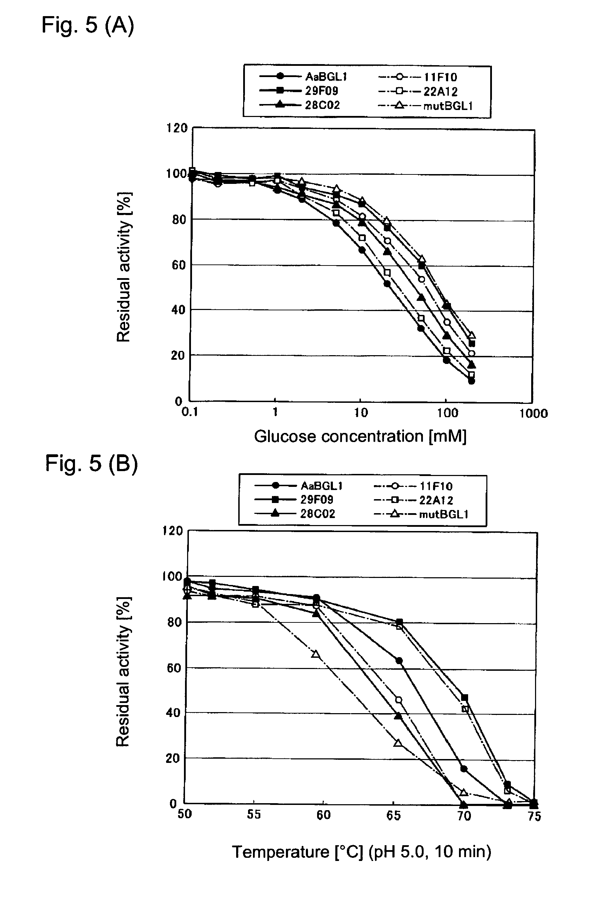

A mutant strain producing wild-type AaBGL1, 11F10 mutant strain, 22A12 mutant strain, 29F09 mutant strain, and 28C02 mutant strain were cultivated, and then the glucose inhibition and the thermostability at a warming temperature of from 50 to 75°C of the culture supernatants of the respective mutant strains were measured. The results are shown in Fig.5. In Fig. 5 (A), the glucosidase activity at a glucose concentration of 0 mM is set to 100%, and the residual glucosidase activities of the each culture supernatant at respective glucose concentrations are shown. In Fig. 5 (B), the glucosidase activity at a condition where preliminary heat treatment is not applied is set to 100%, and the residual glucosidase activities of the each culture supernatant at respective temperature conditions are shown.

### [Example 9]

It was suggested by the results of Examples 1 to 7 that Q142N (substitution of glutamine at position 142 to asparagine), Q156L (substitution of glutamine at position 156 to leucine), I120F (substitution of isoleucine at position 120 to phenylalanine), G298F (substitution of glycine at position 298 to phenylalanine), N458S (substitution of asparagine at position 458 to serine), G468S (substitution of glycine at position 468 to serine), V519A (substitution of valine at position 519 to alanine), and R629L (substitution of arginine at position 629 to leucine) are involved in the resistance to glucose inhibition or the thermostability of AaBGL1. Therefore, with regard to a mutant strain having P3AaBGL1 in which only one of these mutations was introduced, the resistance to glucose inhibition and the thermostability were measured.

### <Preparation of site-specific mutation fragment>

Firstly, mutant strains in which only Q156L, I120F, N458S, or R629L was introduced, were prepared.

Q156L specific amino acid substitution was carried out with primers. By using pSL6P3AaBGL1 as a template, a leu1⁺-Q156L fragment was amplified by PCR method with the primers of SEQ ID NO: 8 and SEQ ID NO: 26. On the other hand, a Q156L-top2 fragment was amplified by PCR method with the primers of SEQ ID NO: 27 and SEQ ID NO: 11.

To a reaction mixture comprised of these two types of fragments, restriction enzyme Dpnl was added to digest the templates, and then the fragments were purified by using SUPREC-PCR for primer substitution. By using 50 ng of the purified two types of fragments, a leu1⁺-Q156L-top2 fragment was amplified by PCR method with the primers of SEQ ID NO: 12 and SEQ ID NO: 13. Thereafter, a column purification was carried out by using SUPREC-PCR.

I120F specific amino acid substitution was carried out with primers. By using pSL6P3AaBGL1 as a template, a leu1⁺-I120F fragment was amplified by PCR method with the primers of SEQ ID NO: 8 and SEQ ID NO: 28. On the other hand, a I120F-top2 fragment was amplified by PCR method with the primers of SEQ ID NO: 29 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-I120F-top2 fragment was amplified by PCR method with the primers of SEQ ID NO: 12 and SEQ ID NO: 13. Thereafter, a column purification was carried out by using SUPREC-PCR.

N458S specific amino acid substitution was carried out with primers. By using pSL6P3AaBGL1 as a template, a leu1⁺-N458S fragment was amplified by PCR method with the primers of SEQ ID NO: 8 and SEQ ID NO: 30. On the other hand, a N458S-top2 fragment was amplified by PCR method with the primers of SEQ ID NO: 31 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-N458S-top2 fragment was amplified by PCR method with the primers of SEQ ID NO: 12 and SEQ ID NO: 13. Thereafter, a column purification was carried out by using SUPREC-PCR.

R629L specific amino acid substitution was carried out with primers. By using pSL6P3AaBGL1 as a template, a leu1⁺-R629L fragment was amplified by PCR method with the primers of SEQ ID NO: 8 and SEQ ID NO: 32. On the other hand, a R629L-top2 fragment was amplified by PCR method with the primers of SEQ ID NO: 33 and SEQ ID NO: 11.

To a reaction mixture comprised of these two types of fragments, restriction enzyme DpnI was added to digest the templates, and then the fragments were purified by using SUPREC-PCR for primer substitution. By using 50 ng of the purified two types of fragments, a leu1⁺-R629L-top2 fragment was amplified by PCR method with the primers of SEQ ID NO: 12 and SEQ ID NO: 13. Thereafter, a column purification was carried out by using SUPREC-PCR.

### <Recovery of mutant strain obtained by Site-Saturation Mutagenesis>

Mutant strains were obtained in the same manner as in Example 1, except that each of the above-prepared fragments was used as a mutant gene-integrated fragment. Then, the nucleotide sequence of P3AaBGL1 gene integrated into the chromosomes of the each mutant strain obtained as above was confirmed.

### <Evaluation of glucosidase activity, resistance to glucose inhibition, and thermostability>

In the same manner as in Example 1, the glucose inhibition and the thermostability at a warming temperature of from 50 to 75°C of the culture supernatants of the respective mutant strains were measured. The results are shown in Table 11.

**TABLE 11**

| | AaBGL1 | Q142N | Q156L | I260F | G298F | N458S | G468S | V519A | R629L | mutBGL1 |
|---|---|---|---|---|---|---|---|---|---|---|
| Activity [mU/ml] | 594 | 533 | 507 | 457 | 278 | 586 | 244 | 426 | 526 | 191 |
| | | | | | | | | | | |

| Glc [mM] | Residual glucosidase activity (%) | | | | | | | | | |
|---|---|---|---|---|---|---|---|---|---|---|
| 0 | 100 | 100 | 100 | 100 | 100 | 100 | 100 | 100 | 100 | 100 |
| 1 | 89.5 | 94.8 | 92.8 | 95.9 | 93.9 | 92.7 | 99.6 | 91.7 | 91.5 | 97.7 |
| 5 | 65.2 | 68.1 | 64.9 | 73.6 | 82.4 | 68.1 | 95.7 | 66.4 | 67.4 | 87.0 |
| 10 | 49.4 | 51.2 | 46.8 | 55.6 | 68.6 | 52.1 | 85.9 | 50.3 | 53.9 | 80.9 |
| 20 | 33.4 | 35.7 | 31.5 | 39.0 | 53.8 | 35.9 | 76.1 | 35.4 | 37.6 | 72.6 |
| 50 | 17.6 | 17.8 | 15.5 | 20.2 | 32.4 | 17.7 | 55.8 | 17.7 | 19.6 | 57.7 |
| 100 | 10.5 | 10.1 | 8.8 | 11.4 | 19.9 | 10.4 | 36.6 | 9.6 | 11.1 | 39.5 |
| 200 | 5.1 | 5.8 | 5.4 | 6.3 | 10.9 | 6.0 | 21.4 | 5.7 | 6.9 | 27.0 |
| | | | | | | | | | | |

| Temp [°C] | Residual glucosidase activity (%) | | | | | | | | | |
|---|---|---|---|---|---|---|---|---|---|---|
| Untreated | 100 | 100 | 100 | 100 | 100 | 100 | 100 | 100 | 100 | 100 |
| 50 | 96.4 | 96.1 | 93.9 | 97.0 | 89.5 | 98.6 | 93.1 | 95.6 | 98.7 | 93.4 |
| 55 | 91.5 | 91.1 | 89.7 | 91.2 | 83.4 | 95.7 | 88.6 | 90.3 | 93.7 | 91.0 |
| 60 | 90.4 | 89.0 | 83.9 | 87.7 | 75.7 | 91.6 | 63.0 | 88.1 | 90.7 | 82.5 |
| 65 | 60.0 | 58.7 | 46.0 | 65.2 | 38.2 | 64.2 | 11.8 | 72.8 | 71.8 | 37.4 |
| 70 | 16.0 | 15.4 | 6.3 | 22.6 | 4.3 | 19.4 | 1.0 | 26.5 | 26.5 | 0.5 |
| 73 | 1.1 | 1.2 | 0.9 | 1.8 | 1.5 | 0.8 | 1.4 | 2.4 | 3.5 | 1.4 |
| 75 | 1.5 | 1.2 | 1.4 | 1.8 | 1.8 | 1.6 | 1.7 | 1.7 | 1.5 | 3.3 |

As a result, I260F was found to increase both the resistance to glucose inhibition and the thermostability of P3AaBGL1. Further, G298F and G468S were found to increase the resistance to glucose inhibition and decrease the thermostability of P3AaBGL1. Further, it was found that the thermostability can be improved without decreasing the resistance to glucose inhibition of P3AaBGL1 by N458S, V519A or R629L. Further, Q156L was found to decrease both the resistance to glucose inhibition and the thermostability of P3AaBGL1.

Among these mutation sites of P3AaBGL1, glycine at position 468 and valine at position 519 are highly preserved in various β-glucosidases. Further, the amino acids correspond to isoleucine at position 260, glycine at position 298, asparagine at position 458 or arginine at position 629 share similar size of side chain and polarity among various β-glucosidases, and the upstream and downstream regions of the amino acids are highly preserved among various β-glucosidases. Accordingly, it is highly probable that all of the above-mentioned amino acids are important for the activity of β-glucosidase, and therefore the activities of various β-glucosidases seemed to be affected in the same manner as P3AaBGL1 described in Examples, by substituting these amino acids in the same manner as for P3AaBGL1.

### [Example 10]

### <Purification of modified-type P3AaBGL1>

11F10 strain, 22A12 strain, 28C02 strain, 29F09 strain and mutBGL1 strain selected from the mutant strains, and ASP3326 strain as the original strain were cultivated in a Sakaguchi flask containing 100 ml of YPD medium at 30°C for two days. To each of the culture supernatants obtained by cultivating them, ammonium acetate was added until 80% saturation was reached, thereby to carry out ammonium sulfate precipitation at 4°C for 2 hours. Thereafter, centrifugation was carried out to collect pellets, and the pellets were dissolved in 10 ml of 10 mM citrate phosphate buffer solution (pH 7.0). Then, desalting and concentration were carried out by using an ultrafiltration membrane having MWCO of 100,000, thereby to recover 1 ml of a crude liquid. Thus recovered crude liquid was purified by using Hitrap QXL column to collect a fraction of a peak of pNPG activity, thereby to obtain a purified sample. Elution was carried out by using a 10 mM citrate phosphate buffer solution (pH 7.0) and a NaCl concentration gradient (0 to 1 M).

### <Characterization of the purified modified-type P3AaBGL1>

In the same manner as in Example 1, the residual glucosidase activities after glucose inhibition at a glucose concentration of from 0.1 to 200 mM were measured. Further, the residual glucosidase activities after heat treatment at a temperature of from 50 to 75°C for 10 minutes were measured. As a result, a glucose inhibition resistance improved-type P3AaBGL1 of 11 F10 strain was found to maintain 53% of the activity in the presence of 50 mM glucose. A thermostability improved-type P3AaBGL1 of 22A12 strain was found to maintain around 80% of the residual activity at 65°C. Further, a glucose inhibition resistance and thermostability improved-type P3AaBGL1 of mutBGL1 strain was found to maintain 60% of the activity in the presence of 50 mM glucose and around 45% of the residual activity at 65°C.

### INDUSTRIAL APPLICABILITY

The β-glucosidase produced from the transformant of the present invention can be used for the enzymatic saccharification of cellulose. Further, when the transformant of the present invention is a transformant which can produce a β-glucosidase having a secretion signal, by cultivating the transformant in a culture broth containing cellulose, it is possible to utilize cellulose contained in the culture broth by the enzymatic saccharification, since the β-glucosidase is secreted into the culture broth.

The entire disclosure of Japanese Patent Application No. 2011-066539 filed on March 24, 2011 including specification, claims, drawings and summary is incorporated herein by reference in its entirety.

## Claims

1. A transformant of a yeast of the genus *Schizosaccharomyces* **characterized by** having a structural gene sequence comprising a region encoding the following modified-type β-glucosidase, and a promoter sequence and a terminator sequence for expressing the structural gene in a chromosome, or alternatively by having the sequences as an extrachromosomal gene.
Modified-type β-glucosidase: A β-glucosidase derived from a filamentous fungus having at least one mutation selected from the group consisting of the following mutations 1 to 6, and has higher thermostability or higher resistance to glucose inhibition than a β-glucosidase which does not have the mutations.
Mutation 1; a mutation in which an amino acid corresponds to isoleucine at position 244 of AaBGL1 (BGL1 of *Aspergillus aculeatus)* is substituted by phenylalanine.
Mutation 2; a mutation in which an amino acid corresponds to glycine at position 282 of AaBGL1 is substituted by phenylalanine.
Mutation 3; a mutation in which an amino acid corresponds to asparagine at position 442 of AaBGL1 is substituted by serine.
Mutation 4; a mutation in which an amino acid corresponds to glycine at position 452 of AaBGL1 is substituted by serine.
Mutation 5; a mutation in which an amino acid corresponds to valine at position 503 of AaBGL1 is substituted by alanine.
Mutation 6; a mutation in which an amino acid corresponds to arginine at position 613 of AaBGL1 is substituted by leucine.

2. The transformant of a yeast of the genus *Schizosaccharomyces* according to Claim 1, wherein the β-glucosidase derived from a filamentous fungus is BGL1.

3. The transformant of a yeast of the genus *Schizosaccharomyces* according to Claim 1 or 2, wherein the filamentous fungus is a microorganism of the genus *Aspergillus.*

4. The transformant of a yeast of the genus *Schizosaccharomyces* according to any one of Claims 1 to 3, wherein the β-glucosidase derived from a filamentous fungus is comprised of an amino acid sequence having at least 70% homology with an amino acid sequence of SEQ ID NO: 1, and is a protein has a catalytic activity to hydrolyze a β-D-glucopyranoside bond.

5. The transformant of a yeast of the genus *Schizosaccharomyces* according to any one of Claims 1 to 4, wherein the structural gene sequence further comprises a region encoding a signal or a region encoding a tag at the 5' end side or the 3' end side of the region encoding the modified-type β-glucosidase.

6. The transformant of a yeast of the genus *Schizosaccharomyces* according to any one of Claims 1 to 5, wherein the structural gene sequence further comprises a region encoding a secretion signal at the 5' end side of the region encoding the modified-type β-glucosidase.

7. A transformation method for a yeast of the genus *Schizosaccharomyces,* **characterized in that** the yeast of the genus *Schizosaccharomyces* is transformed by using a vector containing a structural gene sequence comprising a region encoding the following modified-type β-glucosidase, and a promoter sequence and a terminator sequence for expressing the structural gene.
Modified-type β-glucosidase: A β-glucosidase derived from a filamentous fungus having at least one mutation selected from the group consisting of the following mutations 1 to 6, and has higher thermostability or higher resistance to glucose inhibition than a β-glucosidase which does not have the mutations.
Mutation 1; a mutation in which an amino acid corresponds to isoleucine at position 244 of AaBGL1 (BGL1 of *Aspergillus aculeatus)* is substituted by phenylalanine.
Mutation 2; a mutation in which an amino acid corresponds to glycine at position 282 of AaBGL1 is substituted by phenylalanine.
Mutation 3; a mutation in which an amino acid corresponds to asparagine at position 442 of AaBGL1 is substituted by serine.
Mutation 4; a mutation in which an amino acid corresponds to glycine at position 452 of AaBGL1 is substituted by serine.
Mutation 5; a mutation in which an amino acid corresponds to valine at position 503 of AaBGL1 is substituted by alanine.
Mutation 6; a mutation in which an amino acid corresponds to arginine at position 613 of AaBGL1 is substituted by leucine.

8. The transformation method for a yeast of the genus *Schizosaccharomyces* according to Claim 7, wherein the structural gene sequence further comprises a region encoding a signal or a region encoding a tag at the 5' end side or the 3' end side of the region encoding the modified-type β-glucosidase.

9. The transformation method for a yeast of the genus *Schizosaccharomyces* according to Claim 7, wherein the structural gene sequence further comprises a region encoding a secretion signal at the 5' end side of the region encoding the modified-type β-glucosidase.

10. The transformation method for a yeast of the genus *Schizosaccharomyces* according to any one of Claims 7 to 9, wherein the vector is integrated into at least one position of a chromosome of the yeast of the genus *Schizosaccharomyces.*

11. The transformation method for a yeast of the genus *Schizosaccharomyces* according to Claim 10, wherein the vector is integrated into a transposon gene Tf2 site of the chromosome.

12. A method for producing a β-glucosidase, **characterized in that** the modified-type β-glucosidase is recovered from cells obtained by cultivating the transformant as defined in any one of Claims 1 to 5.

13. A method for producing a β-glucosidase, **characterized in that** the modified-type β-glucosidase is recovered from a culture supernatant obtained by cultivating the transformant as defined in Claim 6.

14. A cellulose degradation method, **characterized in that** the modified-type β-glucosidase obtained by the production method as defined in Claim 12 or 13 is used.

15. A cellulose degradation method, **characterized in that** the transformant as defined in Claim 6 is cultivated in the presence of cellulose.
